# EUROPEAN PATENT APPLICATION

(11) **EP 2 578 588 A1**
(43) Date of publication of application: **10.04.2013**
(21) Application number: 11792009.0
(22) Date of filing: 03.06.2011
(51) Int. Cl.: C07D 487/04, A61K 31/551

(54) **NOVEL 1,4-DIAZEPAM PDE-5 INHIBITOR DERIVATIVES**

(30) Priority: 07.06.2010 MX MX10006227
(71) Applicant: World-Trade Import-Export Wtie, AG, 6302 Zug (CH)
(72) Inventor: GARCÍA ARMENTA, María Elena, 45020 Guadalajara Jalisco (MX); ALVAREZ OCHOA, Víctor Guillermo, 45222 Zapopan Jalisco (MX); OSNAYA MORALES, Roberto Carlos, 52918 Atizapán de Zaragoza Estato de México (MX); ZARATE BARAJAS, Erika Alejandra, 45418 Tonalá Jalisco (MX)
(74) Representative: Carpintero Lopez, Francisco
(86) International application number: PCT/IB2011/001228
(87) International publication number: WO 2011/154798

(57) **Abstract**

It describes a compound of general formula I, 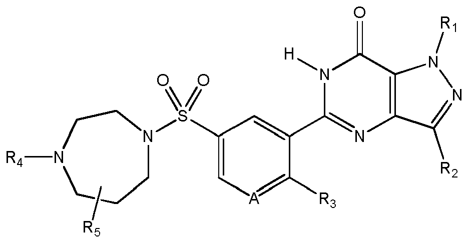 methods for its preparation, pharmaceutical compositions containing it and the use of the latter in the treatment of erectile dysfunction.

## Description

### FIELD OF THE INVENTION.

The present invention relates to the synthesis, preparation, procedures and use of new compounds derived from 1,4-diazepane that act as inhibitors of the phosphodiesterase enzyme type 5 (PDE-5); said compounds are appropriate for the manufacture of a medicament useful in the treatment of erectile dysfunction.

### BACKGROUND OF THE INVENTION.

Erectile dysfunction is defined as the persistent inability to maintain an erection of the penis of sufficient stiffness to have satisfactory sexual activity. It has been calculated that this ailment is suffered by approximately 20 to 25 million men worldwide.

There are different therapeutic alternatives for treating erectile dysfunction. For example, there are PDE-5 enzyme blockers, which are specific to the active site of cyclic GMP, which work by increasing levels of cGMP, leading to longer penis erection times enough to allow satisfactory sexual relations.

One example of this type of blockers is sildenafil. Several processes have been described for manufacture sildenafil. For example, patent EP 0463756 describes its preparation by a coupling reaction between 1-methylpiperazine and a chlorosulfonated derivate **A** (see **Scheme 1).**

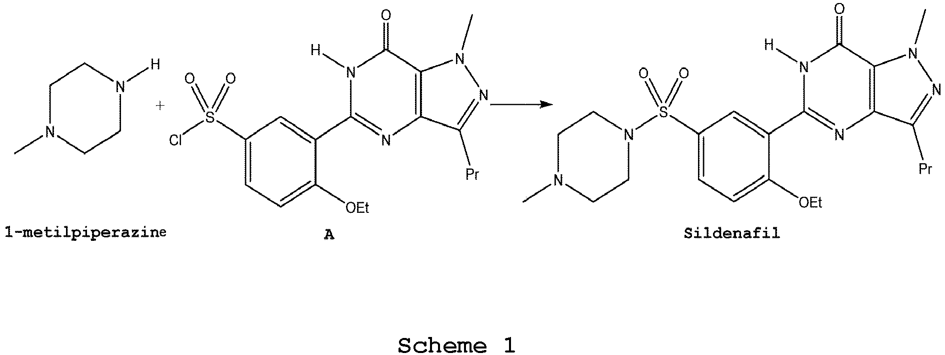

Patent EP 1077214 describes the synthesis of sildenafil by coupling between a chlorosulfonated piperazine **B** and the intermediate **C** shown in Scheme 2.

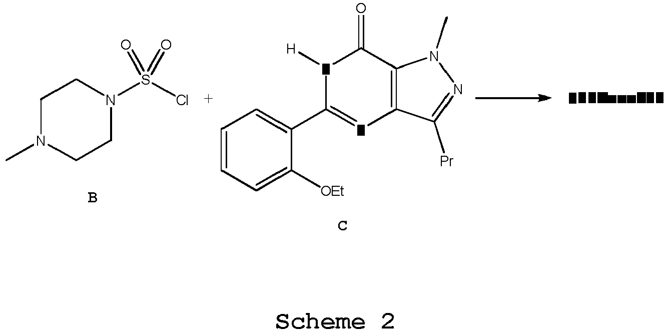

Other processes are described in patents EP 812845 and EP 1002798, which are based on closing the ring of derivate D to form the pyrazolopyrimidinone system.

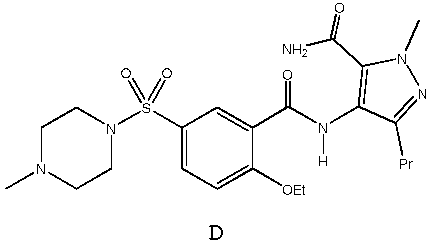

International patent application WO 01/98284 describes the formation of sildenafil by coupling between the derivate E and the pyrazole derivate F (Scheme 3).

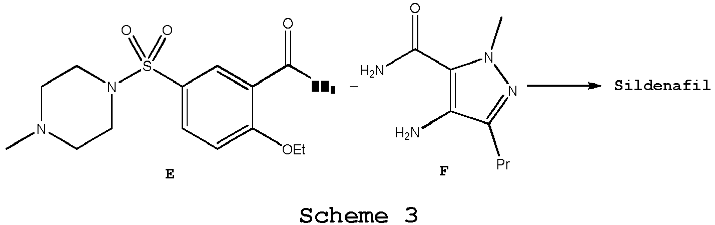

Patent application MX PA99010322 and document WO 01/19827 relate to a process for reductive alkylation of the derivate G, wherein R₁= H or Me and/or R₂= H or Me.

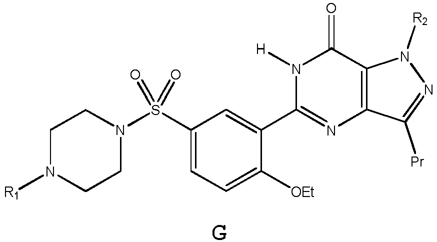

Patent US 6204383 describes the preparation of sildenafil by closing the piperazine ring of the derivate H.

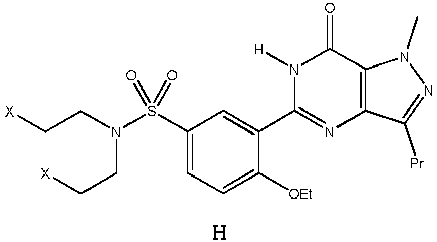

Patent application WO 2008/152177 describes the preparation of sildenafil by a coupling reaction between the boronic derivate I and the derivate J, performed under Suzuki conditions (Scheme 4).

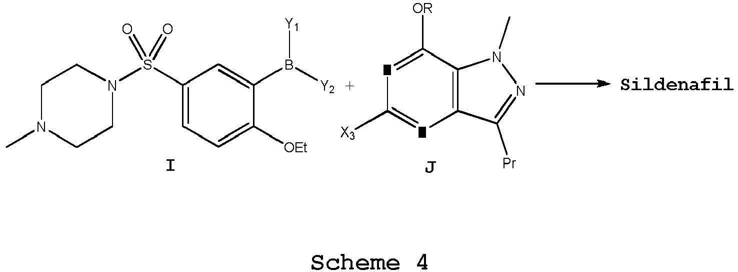

In addition, modifications have been suggested in some functional groups of the base structure of sildenafil, indicated in Scheme 5.

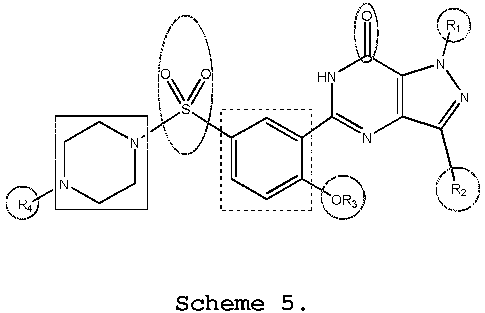

For example, in alkyl groups R₁, R₂, R₃ and R₄ (EP 0463756; Terrett, N. K. et al., Bioorg. Med. Chem. Lett., 6, 1819-1824, (1996*) ;* Kim, D.-K. et al., Bioorg. Med. Chem., 9, 3013-3021, (2001*);* Zhao, Y. -F. et al., Chem. Res. Chinese U., 22, 468-473, (2006*) ;* Flores, H. A. et al., J. Med. Chem., 51, 2807-2815, (2008*)*)*.*

International patent application WO 00/24745 describes changes in the position of substituent R₁ from position 1 to position 2 of the fragment pyrazole[4,3-*d*]pyrimidinone, giving rise to another family of compounds.

Another functional group that has been modified is the phenyl group, for other rings such as chromane (Kim, D.-K. et al., Bioorg. Med. Chem., 9, 1609-1616, (2001*)*)*,* and for heterocycles, such as pyridine (WO 01/98284; WO 99/54333), thiophene, benzo[d]oxazol, benzo[d]isoxazol, benzo[*d*][1,3]dioxol (WO 01/03644) and benzofurane *(*Nader R. Al-bojuk, et al., Heterocycles, 55, 1789-1804, (2001*)).*

The oxygen in the carbonyl present in the amide has also been replaced with sulfur and thioanalogues (Kim, D.-K. et al., Bioorg. Med. Chem. Lett., 14, 2099-2103, (2004*);* P. Zou et al. J. Pharm. Biomed. Anal., 47, 279-284, (2008*)).*

In addition, the sulphonyl group has been substituted with the acetyl group *(*P. Zou et al., J. Chrom. A, 1104, 113-122, (2006*).* The *N*-oxide derivates of sildenafil have also been prepared (WO 2009/000798).

It is especially interesting to point out that modifications have also been made on the piperazine ring presenting the molecular structure of sildenafil. For example, it has been replaced by cyclic amines such as: pyrrolidine, piperidine, morfoline (WO 01/03644, US 5250534); cyclic amines such as: substituted ethylenediamine (Flores Toque, H. A., et al., J. Med. Chem., 51, 2807-2815, (2008*)),* derivates of methyleneamines, hydroxylamine, mercaptoethanol and derivates of hydrazine *(*Khan, K. M. et al. Mol. Divers., 9, 15-26, (2005*)*)*.*

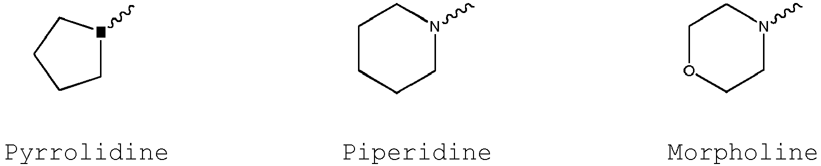

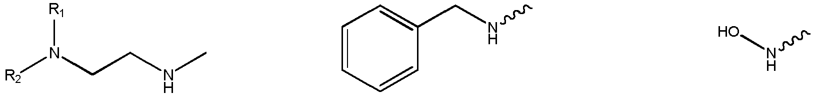

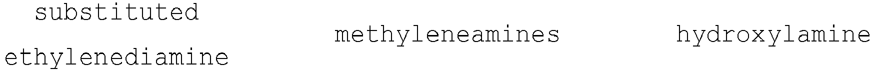

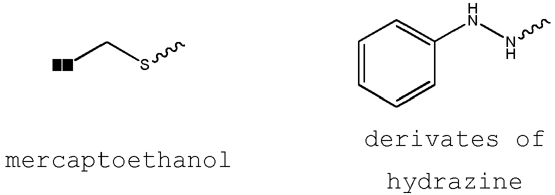

However, despite the satisfactory therapeutic efficacy that sildenafil has shown in the treatment of erectile dysfunction, it is known that due to its relative selectivity versus enzymes of the PDE type, sildenafil causes clinical significant undesirable adverse effects, such as headaches, nausea, rubefaction, numbness of the limbs, dyspepsia, fuzzy vision and photophobia.

For this reason, there remains a need to design and synthesize new compounds showing better selectivity for PDE-5 than for other PDE type enzymes, such as PDE-6, a greater therapeutic efficacy and safety, and in addition allow reducing adverse effects associated with consumption.

### BRIEF DESCRIPTION OF THE INVENTION

In light of the drawbacks of the prior art, the present invention relates to a compound of general formula **(I)** 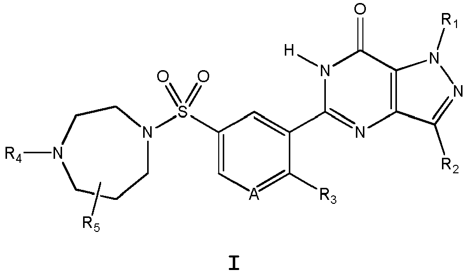 wherein:
- A: represents a carbon or nitrogen atom;
- R₁: represents a proton, a linear or branched chain C₁-C₆ alkyl, a C₃-C₆ cycloalkyl, a COR₆ group, a derivate of carboxylic acid of C₁-C₈, a heterocyclic system with one ring or fused rings with three to six members in each ring, with one to five heteroatoms;
- R₂: represents a proton, a linear or branched chain C₁-C₆ alkyl, a C₃-C₆ cycloalkyl or a C₁-C₃alkoxyl;
- R₃: represents a proton, a linear or branched chain C₁-C₆ alkyl, a hydroxyl, a C₁-C₃ alkoxyl, optionally substituted, a halogen, a heterocyclic system with a single ring or fused rings with three to six members in each ring, with one to five heteroatoms;
- R₄: represents a proton, a linear or branched chain C₁-C₈ alkyl optionally with a hydroxyl group at the end of the chain, a C₃-C₆ cycloalkyl optionally with a hydroxyl group in the ring, a group derived from COR₇, a group derived from CO₂R₈, a derivate of carboxylic acid with C₁-C₈ that can be directly linked to the nitrogen of the 1,4-diazepane or preceded by one, two or three units of methylene, or an amine protector group such as Fmoc (9-fluorenylmethoxycarbonyl), 2,2,2-trichloroethyl carbamate, Trt (triphenylmethyl), Teoc (2-(trimethylsiliethoxyl), Boc (*t*-butoxycarbonyl), Alloc (vinylmethoxycarbonyl), Nps (2-nitrophenylsulphenyl), CBz (benzyloxycarbonyl), *m*-nitrophenyl carbamate, Dts (dithiasuccinoyl), Tos (*p*-toluensulfonyl), Tf (trifluoromethanesulfone), Ses (2-(trimethylsilyl)ethanesulfonyl) or Bpoc (2-(4-biphenyl)-isopropoxycarbonyl);
- R₅: represents a proton, a linear or branched chain C₁-C₆ alkyl, a functional group such as hydroxyl, methoxyl, or carboxylic acid;
- R₆: represents a proton or a linear or branched chain C₁-C₆ alkyl;
- R₇: represents a proton, a linear or branched chain C₁-C₆ alkyl, a C₃-C₆ cycloalkyl where it can be a saturated or aromatic ring, and can also contain a partially or totally saturated or unsaturated heterocycle;
- R₈: represents a proton, a linear or branched chain C₁-C₆ alkyl, a C₃-C₆ cycloalkyl where the ring can be saturated or aromatic, and can also contain groups such as benzyl or methylfluorenyl;
and their salts and N-oxide compounds.

Specifically, it can be seen that the compound of general formula **I** is structurally characterized by replacing the piperazine ring with 1,4-diazepane derivates, which act as PDE-5 enzyme inhibitors; therefore, these compounds are suitable for manufacture a medicament useful in the treatment of erectile dysfunction.

1,4-diazepane derivates, also known as homopiperazine derivates, give the compound of general formula **I** fewer symmetry elements with respect to the structure of sildenafil, thereby conferring them with different physico-chemical properties, such as improved solubility.

The salts of the new compound of the present invention have an improved solubility with respect to sildenafil citrate (3.5 mg/mL), which together with the satisfactory permeability of the compounds, lead to improved therapeutic effects for the treatment of erectile dysfunction, also achieving to reduce the adverse effects related to its use.

The present invention also relates to the methods used to obtain the compound of general formula **I,** characterized by the Kharasch and Negishi coupling reactions of intermediate **III'** with the compounds of general formula **IV, VIII** and **IX** to form the compounds of general formula **V', VI'** and **I'**, respectively (see **Scheme 6).**

The intermediate **V'** is chlorosulfonated to obtain the intermediate **VI',** which in turn is coupled to the 1,4-diazepane derivates of general formula **VII** to obtain the compound of general formula **I'** that is in tautomeric equilibrium with the compound of general formula **I.**

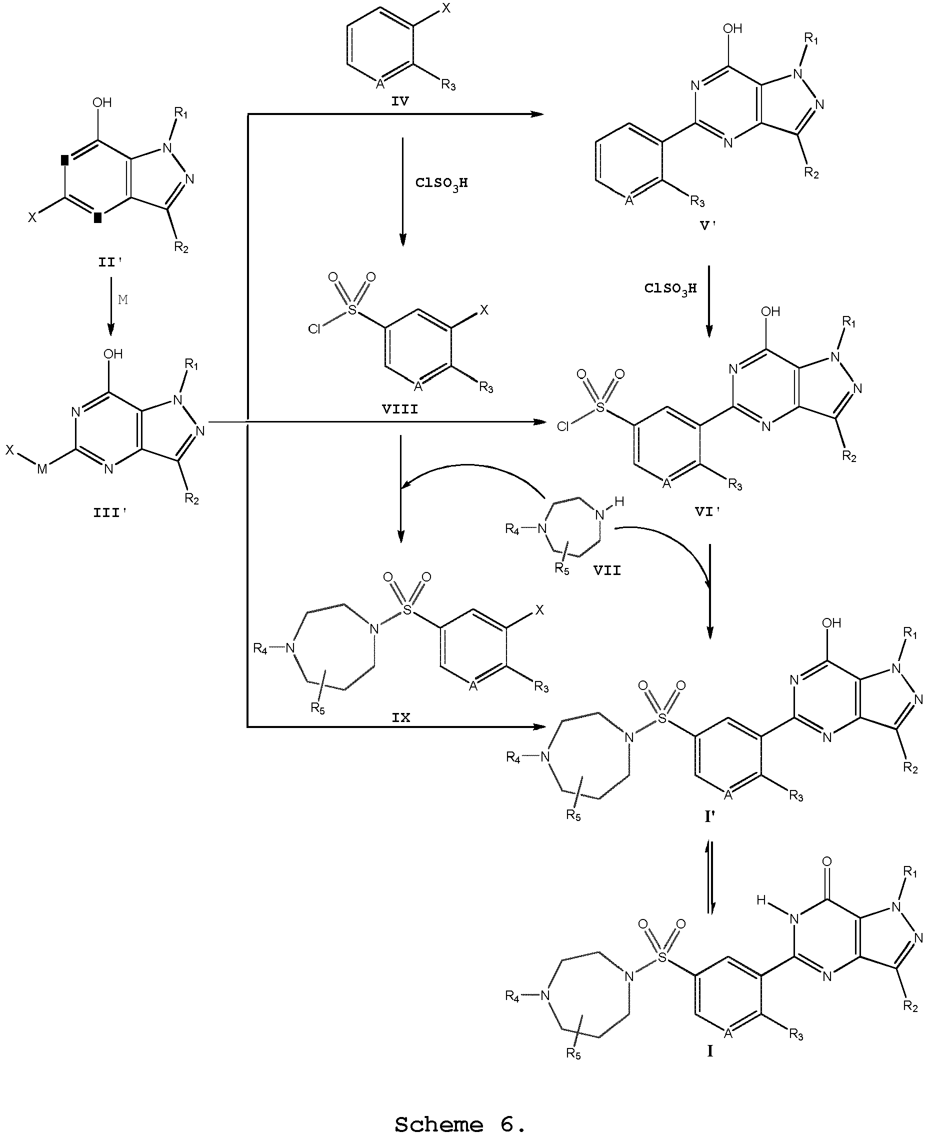

In another aspect of the invention, a pharmaceutical composition of the invention is provided that comprises the compound of general formula **I** and a coadjuvant agent, diluent agent and/or a pharmaceutically acceptable carrier.

Moreover, the invention relates to a method for treatment that consists in administering a therapeutically effective amount of the compound of general formula **I** to a mammal to treat erectile dysfunction.

In addition, the use of the compound of general formula **I** to prepare a medicament useful in the treatment of erectile dysfunction is described.

### BRIEF DESCRIPTION OF THE DRAWINGS

**Figure 1** is an infrared absorption spectrum for 5-(5-(1,4-diazepane-1-ylsulfonyl)-2-ethoxyphenyl)-1-methyl-3-propyl-1*H*-pyrazolo[4,3-*d*]pyrimidin-7(6*H*)-one (Compound 1).
**Figure 2** is a proton nuclear magnetic resonance spectrum (200 MHz) for 5-(5-(1,4-diazepane-1-ylsulfonyl)-2-ethoxyphenyl)-1-methyl-3-propyl-1*H*-pyrazolo[4,3-*d*]pyrimidin-7(6H)-one (Compound **1).**
**Figure 3** is an electrospray ionisation mass spectrum for 5-(5-(1,4-diazepane-1-ylsulfonyl)-2-ethoxyphenyl)-1-methyl-3-propyl-1*H*-pyrazolo[4,3-*d*]pyrimidin-7(6*H*)-one (Compound **1).**
**Figure 4** is an infrared mass absorption spectrum for tert-butyl 1,4-diazepane-1-carboxylate.
**Figure 5** is an infrared absorption spectrum for 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 6** is a proton nuclear magnetic resonance spectrum (200MHz) for 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 7** is an electrospray ionisation mass spectrum for 5-(2-ethoxy-5-(4-methyl-1,4-diazepane-1-ylsulfonyl)phenyl)-1-methyl-3-propyl-1*H*-pyrazolo[4,3-d]pyrimidin-7(6*H*)-one (Compound **3).**
**Figure 8** is an infrared absorption spectrum for the citrate salt of 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 9** is a proton nuclear magnetic resonance spectrum (500 MHz) for the citrate salt of 5-(2-ethoxy-5-(4-methyl-1,4-diazepane-1-ylsulfonyl)phenyl)-1-methyl-3-propyl-1*H-*pyrazolo[4,3-*d*]pyrimidin-7(6*H*)-one (Compound **3).**
**Figure 10** are thermographs of the compound **3,** citric acid and the citrate salt of the same compound.
**Figure 11** is an infrared absorption spectrum for the chlorohydrate salt of 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 12** are thermographs of the compound **3,** and the chlorohydrate salt of the same compound.
**Figure 13** is an infrared absorption spectrum for the tartrate salt of 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 14** is an infrared absorption spectrum for the N-oxide compound of 5-(2-ethoxy-5-(4-methyl-1,4-diazepane-1-ylsulfonyl)phenyl)-1-methyl-3-propyl-1*H*-pyrazolo[4,3-*d*]pyrimidin-7(6*H*)-one (Compound **3).**
**Figure 15** is a graph comparing the inotropic effect of sildenafil and of compound **3** on the left ventricle of an isolated rat's heart, with a perfusion pressure of 140 mmHg.

### DETAILED DESCRIPTION OF THE INVENTION.

The present invention relates to the development of the compound with general formula **I** containing in its chemical structure the basic skeleton of pyrazolo[4,3-d]pyrimidin-7-ones, characterized by having attached at position five a fragment of substituted arylsulphonyl-1,4-diazepanyl.

Thus, in a first aspect of the present invention, the compound of general formula I is provided 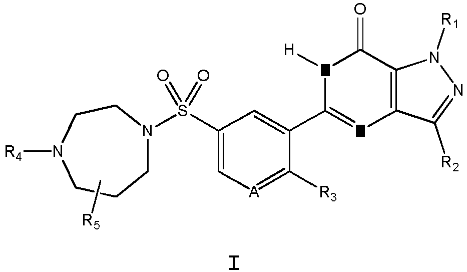 wherein:
- A: represents a carbon or nitrogen atom;
- R₁: represents a proton, a linear or branched chain C₁-C₆ alkyl, a C₃-C₆ cycloalkyl, a COR₆ group, a derivate of carboxylic acid of C₁-C₈, a heterocyclic system with one ring or fused rings with three to six members in each ring, with one to five heteroatoms;
- R₂: represents a proton, a linear or branched chain C₁-C₆ alkyl, a C₃-C₆ cycloalkyl or a C₁-C₃alkoxyl;
- R₃: represents a proton, a linear or branched chain C₁-C₆ alkyl, a hydroxyl, a C₁-C₃ alkoxyl, optionally substituted, a halogen, a heterocyclic system with a single ring or fused rings with three to six members in each ring, with one to five heteroatoms;
- R₄: represents a proton, a linear or branched chain C₁-C₈ alkyl optionally with a hydroxyl group at the end of the chain, a C₃-C₆ cycloalkyl optionally with a hydroxyl group in the ring, a group derived from COR₇, a group derived from CO₂R₈, a derivate of carboxylic acid with C₁-C₈ that can be directly linked to the nitrogen of the 1,4-diazepane or preceded by one, two or three units of methylene, or an amine protector group such as Fmoc (9-fluorenylmethoxycarbonyl), 2,2,2-trichloroethyl carbamate, Trt (triphenylmethyl), Teoc (2-(trimethylsiliethoxyl), Boc (t-butoxycarbonyl), Alloc (vinylmethoxycarbonyl), Nps (2-nitrophenylsulphenyl), CBz (benzyloxycarbonyl), m-nitrophenyl carbamate, Dts (dithiasuccinoyl), Tos (p-toluensulfonyl), Tf (trifluoromethanesulfone), Ses (2-(trimethylsilyl)ethanesulfonyl) or Bpoc (2-(4-biphenyl)-isopropoxycarbonyl);
- R₅: represents a proton, a linear or branched chain C₁-C₆ alkyl, a functional group such as hydroxyl, methoxyl, or carboxylic acid;
- R₆: represents a proton or a linear or branched chain C₁-C₆ alkyl;
- R₇: represents a proton, a linear or branched chain C₁-C₆ alkyl, a C₃-C₆ cycloalkyl where it can be a saturated or aromatic ring, and can also contain a partially or totally saturated or unsaturated heterocycle;
- R₈: represents a proton, a linear or branched chain C₁-C₆ alkyl, a C₃-C₆ cycloalkyl where the ring can be saturated or aromatic, and can also contain groups such as benzyl or methylfluorenyl;
and its salts and *N*-oxide compounds.

The substituent R₅ can confer an asymmetrical center to the compound of general formula **I,** so that in accordance with the present invention, the compound of general formula **I** can exist as stereoisomers. In accordance with the present invention, both racemic mixtures and individually separated stereoisomers are included, except when R₅ is a proton. When one or more substituents (R₁, R₂, R₃ and R₄) in the structure of the compound of general formula **I** contain an asymmetry center and R₅ is other than a proton, then the compound of general formula **I** can be found as a mixture of diastereoisomers or as individually separated stereoisomers.

The compound of general formula **I,** in accordance with the present invention, can exist in tautomeric form, comprising both mixtures and individually separated or favoured tautomers.

The compound of general formula **I** can contain in its chemical structure isotopes of deuterium, carbon 13, and/or nitrogen 15, which are suitable for performing pharmacological studies.

The compound of general formula **I,** in accordance with the present invention, can be presented as salts. The physiologically acceptable salts are preferred according to the present invention. The physiologically acceptable salts are formed as an ionic product of the interaction of a pharmaceutically acceptable acid with the basic center of the compound of general formula **I.**

The physiologically acceptable salts of the compound of general formula **I** are formed using inorganic or organic acids. The preferred salts when using inorganic acids are: hydrochloric acid, hydrobromic acid, phosphoric acid or sulfuric acid; the preferred salts using organic acids are those containing functional groups as carboxylic or sulfonic acids, such as: maleic acid, acetic acid, fumaric acid, malic acid, citric acid, tartaric acid, lactic acid, benzoic acid, methanesulfonic acid, ethanesulfonic acid, phenylsulfonic acid or toluenesolufonic acid.

In accordance with the present invention, the physiologically acceptable salts can also be metal or ammonium salts of the compound of general formula **I.** Preferred salts are those of sodium, potassium, magnesium or calcium, as well as ammonium salts derived from ammonia or organic amines, such as: ethylamine, di- or tri-ethylamine, di- or tri-ethanolamine, dicyclohexylamine, arginine, lysine or ethylenediamine.

The acids used for form the pharmaceutically acceptable salts of the compounds of general formula **I,** are used in a ratio of 1:1 to 1:2, with respect to the base compound of general formula **I.**

The base compound of general formula **I** is used in a ratio of 1:1 with the following acids: hydrochloric, hydrobromic, phosphoric, sulfuric, fumaric, lactic, maleic and citric; and in a ration of 1:2 with the acids: maleic, sulfuric, phosphoric, fumaric, tartaric and citric.

A preferred modality of the compound of general formula I, in accordance with the present invention, is that where:
- A: represents a carbon or nitrogen atom;
- R₁: represents a C₁-C₂ alkyl or a derivate of pentanoic acid;
- R₂: represents a linear or branched chain C₁-C₃ alkyl;
- R₃: represents a hydroxyl or alkoxyl group of C₁-C₃ optionally substituted;
- R₄: represents a proton, a C₁-C₃ alkyl group optionally substituted with a hydroxyl group at the end of the chain, a COR₇ group or an amine protector group, such as: Fmoc, Trt, Teoc, Boc, Nps, Cbz, Dts, Tos, Tf, Ses or Bpoc;
- R₅: represents a proton or a C₁-C₃ alkyl group;
- R₇: represents a proton or a methyl;
and its salts and N-oxide compounds.

A more preferred modality of the compound of general formula I, in accordance with the present invention, is that where:
- A: represents a carbon or nitrogen atom;
- R₁: represents methyl or ethyl;
- R₂: represents n-propyl;
- R₃: represents ethoxyl or propoxyl;
- R₄: represents a proton or methyl or ethyl or propyl, optionally substituted with a hydroxyl group, a COR₇ group or an amine protector group, such as: Fmoc and Boc;
- R₅: represents a proton or methyl;
- R₇: represents a proton or methyl;
and its salts and N-oxide compounds.

The especially preferred compounds in accordance with the present invention are those mentioned in **Table 1:**

**Table 1**

| | Structure | Name |
|---|---|---|
| 1 | 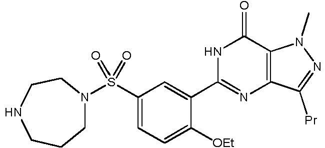 | 5-(5-(1,4-diazepan-1-ylsulfonyl)-2-ethoxiphenyl)-1-methyl-3-propyl-1*H*-pyrazolo[4,3-*d*]pyrimidin-7(6*H*)-one. |
| 2 | 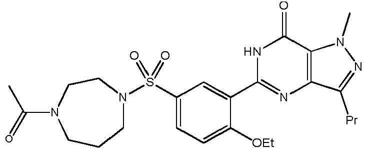 | 5-(5-(4-acetyl-1,4-diazepan-1-ylsulfonyl)-2-ethoxyphenyl)-1-methyl-3-propyl-1*H*-pyrazolo[4,3-*d*]pyrimidin-7(6*H*)-one. |
| 3 | 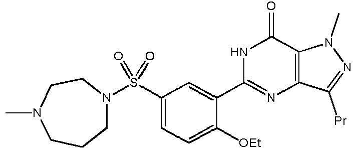 | 5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one. |
| 4 | 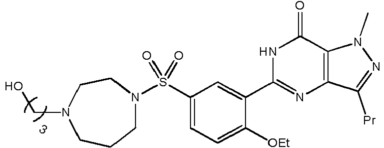 | 5-(2-ethoxy-5-(4-(2-hydroxypropyl)-1,4-diazepan-1-ylsulfonyl)phenyl)-1-methyl-3-propyl-1*H*-pyrazolo[4,3-*d*]pyrimidin-7(6*H*)-one. |
| 5 | 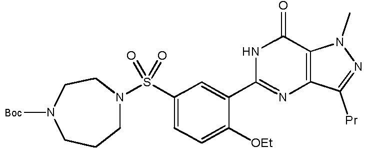 | tert-butyl 4-(4-ethoxy-3-(1-methyl-7-oxo-3-propyl-6,7-dihydro-1*H*-pyrazolo[4,3-*d*]pyrimidin-5-yl) phenylsulfonyl)-1,4-diazepane-1-carboxylate. |
| 6 | 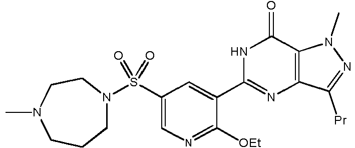 | 5-(2-ethoxy-5-(4-methyl-1,4-diazepan-1-ylsulfonyl)pyridin-3-yl)-1-methyl-3-propyl-1*H*-pyrazolo[4,3-d] pyrimidin-7(6H)-one. |
| 7 | 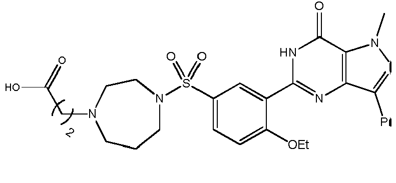 | 2-(4-(4-ethoxy-3-(1-methyl-7-oxo-3-propyl-6,7-dihydro-1*H*-pyrazolo[4,3-d]pyrimidin-5-yl) phenylsulfonyl)-1,4-diazepan-1-yl) propionic acid. |

### Preparation Methods.

In addition to the above, and according to an additional aspect of the present invention, a method is provided for the preparation of the compound of general formula **I** characterized by the formation of intermediaries through Kharasch and Negishi type coupling processes, and by several common reactions such as chlorosulfonation and couplings for the formation of the N-S bond.

In the method, the compound of general formula **I** can be prepared reacting a compound of general formula **II'** 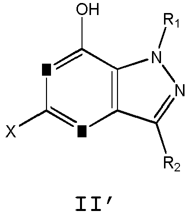 wherein:
R₁ and R₂ have been defined previously for the compound of general formula **I,** and X represents a halogen atom, such as fluorine, chlorine, bromine or iodine, with a metal such as magnesium or with zinc chloride (ZnCl₂), to form the compound of general formula **III'** 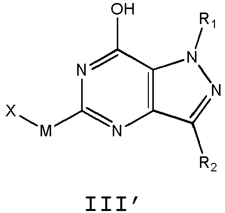 wherein:
R₁, R₂ and X have the meaning given above, and M represents an atom of magnesium or zinc.

Subsequently a coupling reaction is performed between the compound of general formula **III'** and the aryl halide of general formula **IV** 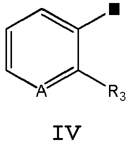 wherein:
A and R₃ are as defined previously for the compound of general formula **I,** and X represents a halogen atom such as fluorine, chlorine, bromine or iodine, in the presence of a suitable catalyst, such as:
   Ni(dppp)Cl₂ or Ni(PPh₃)₂Cl₂ complexes (Kharasch coupling conditions) for the case of the compound of general formula **III'** wherein M is an atom of magnesium, or
   metallic complexes of triphenylphosphines of palladium or nickel (Negishi coupling conditions) for the case of the compound of general formula **III'** wherein M is an atom of zinc,
   to form the compound of general formula **V'** 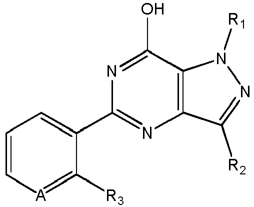 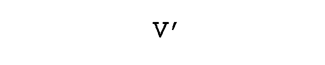
wherein:
A, R₁, R₂ and R₃ represent functional groups that have been defined previously for the compound of general formula **I.**

Then the compound of general formula **V'** is reacted with chlorosulfonic acid (ClSO₃H) in the presence of inert solvents and a suitable base, to form the compound of general formula **VI'** 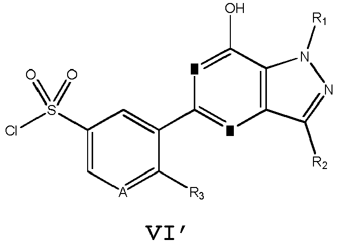 wherein:
A, R₁, R₂ and R₃ have been defined previously for the compound of general formula **I.**

Finally, the compound of general formula **VI'** is reacted with 1,4-diazepane derivates of general formula **VII** 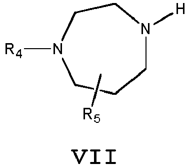 wherein:
R₄ and R₅ have been defined previously for the compound of general formula **I,**
in the presence of a solvent and a suitable base, this stage preferably is performed at ambient temperature or low temperatures, such as from 3 to 13 °C, to form the compound of general formula **I',** 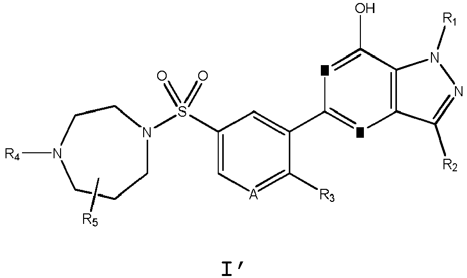 wherein:
   A, R₁, R₂, R₃ R₄ and R₅ represent groups that have been defined previously for the compound of general formula **I.**

The compound of general formula **I'** is in an iminol-amide tautomeric equilibrium with the compound of general formula **I,** so that the compounds of general formula **I'** and **I** can be found in both mixtures and in the form of tautomers individually separated or favoured.

According to the present invention, the procedure described above can be explained, by way of example, with the following reaction stages:
*Stage* A: Metalation of the compound of general formula **II'.** 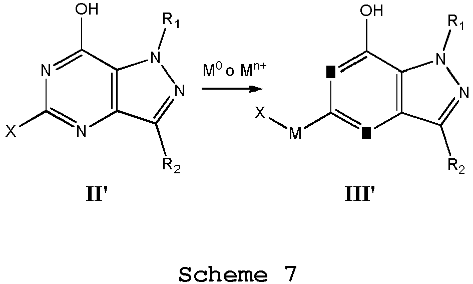
*Stage B:* Formation of the C-C bond by the Kharasch (M=Mg) and Negishi (M=Zn) type coupling reactions between the compounds of general formula **III'** and IV. 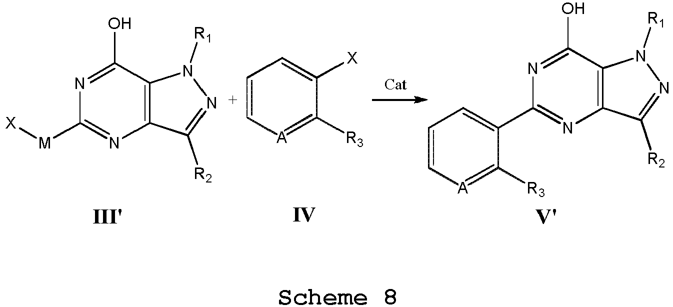
*Stage* C: Chlorosulfonation of the compound of general formula **V'.** 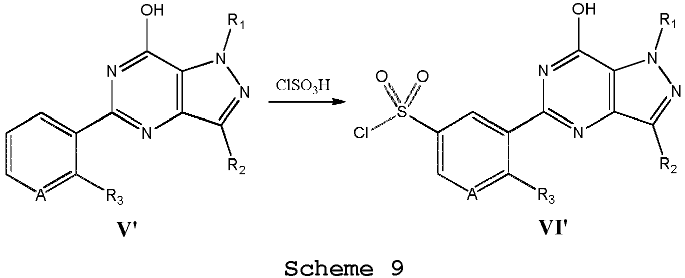
*Stage* D: Coupling of the compound of general formula **VI'** and the 1,4-diazepane derivate of general formula **VII.**

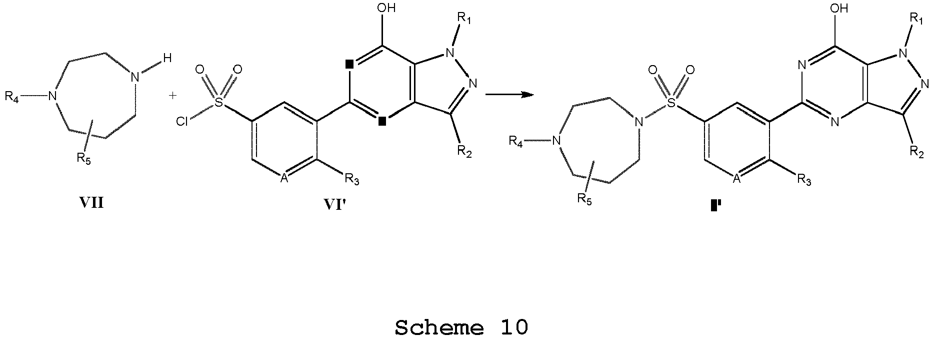

Finally, an iminol-amide tautomeric equilibrium is established between the compounds of general formula **I'-I.**

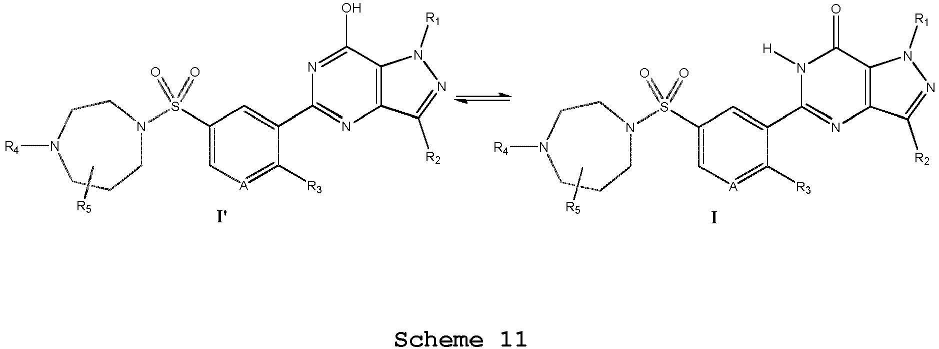

The compound of general formula **I,** according to the present invention, can also be prepared by an alternative manner in which the compound of general formula **III'** is reacted, obtained by the metalation process of the compound of general formula **II',** as indicated in Stage A of the method described above, 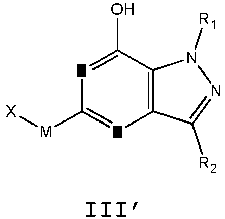 wherein:
R₁ and R₂ have the meaning given above for the compound of general formula **I,** X represents a halogen atom, such as fluorine, chlorine, bromine or iodine, and M represents an atom of magnesium or zinc,
with a chlorosulfonated aryl halide with general formula **VIII,** obtained by chlorosulfonation of the compound of general formula **IV,** 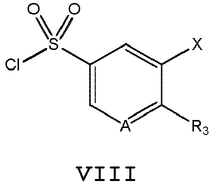 wherein:
   A and R₃ ave been defined previously for the compound of general formula **I,** and X has the meaning given above,
      reacting in the presence of a suitable catalyst such as: Ni(dppp)Cl₂ or Ni(PPh₃)₂Cl₂ complexes (Kharasch coupling conditions) for the case of the compound of general formula **III'** where M is an atom of magnesium, or
   metallic complexes of triphenylphosphines of palladium or nickel (Negishi coupling conditions) for the case of the compound of general formula **III'** where M is an atom of zinc, to form the compound of general formula **VI'** 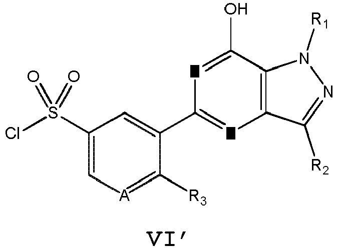
wherein:
A, R₁, R₂ and R₃ represent functional groups that have been defined previously for the compound of general formula **I.**

Then, the compound of general formula **VI'** is reacted with 1,4-diazepane derivates of general formula **VII,** 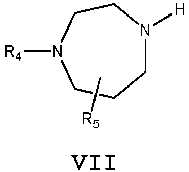 wherein:
R₄ and R₅ have been defined previously for the compound of general formula **I,**
in the presence of a solvent and a suitable base, this stage preferably performed at ambient temperature or low temperatures, such as from 3 to 13 °C, to form the compound of general formula **I',** 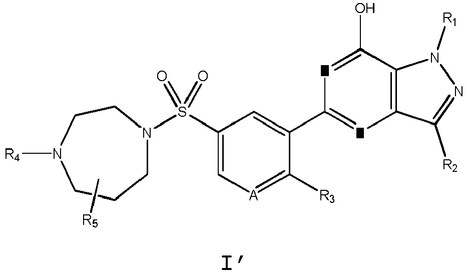
wherein:
A, R₁, R₂, R₃ R₄ and R₅ represent groups that have been defined previously for the compound of general formula **I..**

The compound of general formula **I'** is in an iminol-amide tautomeric equilibrium with the compound of general formula **I,** so that the compounds of general formula **I'** and **I** can be found in both mixtures and in the form of tautomers individually separated or favoured.

According to the present invention, the modality of the method described above can be clarified, by way of example, with the following reaction stages:
*Stage* A: Formation of the C-C bond by the Kharasch (M=Mg) and Negishi (M=Zn) type coupling reactions between the compounds of general formula **VIII** and **III'.** 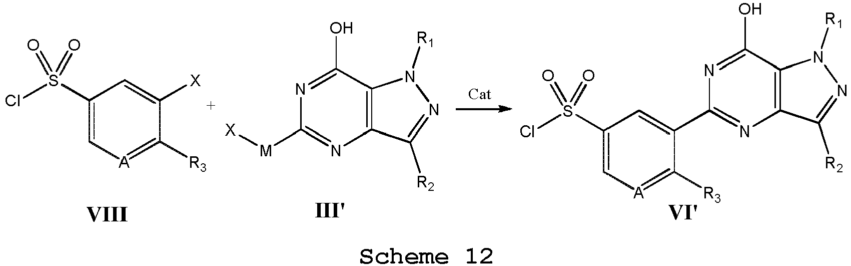
*Stage B:* Coupling of the compound of general formula **VI'** and thel,4-diazepane derivate of general formula **VII.** 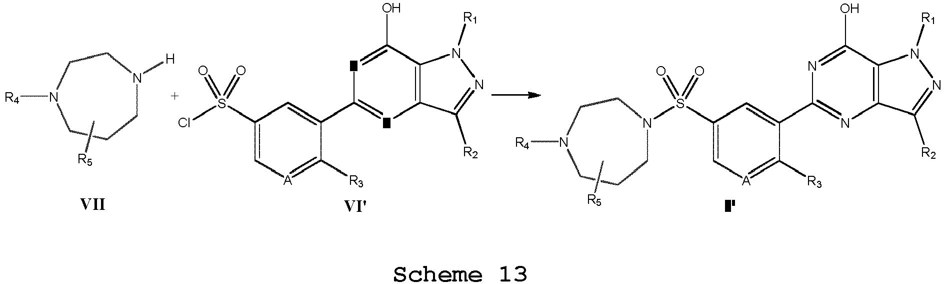
Finally, an iminol-amide tautomeric equilibrium is established between the compounds of general formula **I'-I,** as described in **Scheme 11** of the previous method.

The compound of general formula **I,** according to the present invention, can also be prepared by an additional manner by reacting a compound of general formula **III',** obtained by the metalation process of the compound of general formula **II',** as indicated in the methods described above, 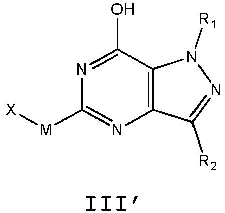 wherein:
R₁ and R₂ have the meaning given above for the compound of general formula **I,** X represents a halogen atom, such as fluorine, chlorine, bromine or iodine, and M represents an atom of magnesium or zinc,
with the arylhalogenated sulfonamide of general formula **IX,** obtained by the reaction between the 1,4-diazepane derivate of general formula **VII** and the chlorosulfonated derivate of general formula **VIII,** 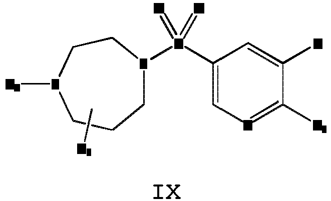
wherein:
A, R₃, R₄ and R₅ have been defined previously for the compound of general formula **I,** and X has the meaning given above,

The reaction is performed in the presence of a suitable catalyst, such as:
Ni(dppp)Cl₂ or Ni(PPh₃)₂Cl₂ complexes (Kharasch coupling conditions) for the case of the compound of general formula **III'** where M is an atom of magnesium, or
metallic complexes of triphenylphosphines of palladium or nickel (Negishi coupling conditions) for the case of the compound of general formula **III'** where M is an atom of zinc,
to form the compound of general formula **I'** 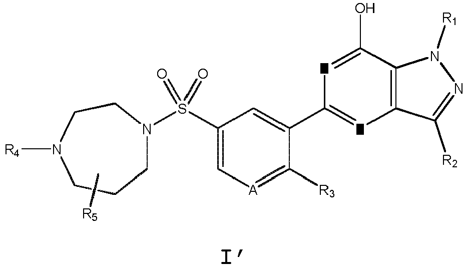
wherein:
A, R₁, R₂, R₃, R₄ and R₅ represent functional groups that have been defined previously for the compound of general formula **I.**

According to the present invention, the compound of general formula **I'** is in iminol-amide tautomeric equilibrium with the compound of general formula **I,** so that the compounds of general formula **I** and **I'** can be found in both mixtures and in the form of tautomers individually separated or favoured.

According to the present invention, the modality of the method described above can be explained, by way of example, with the following reaction stages:
*Stage* A: Formation of the C-C bond by the Kharasch (M=Mg) and Negishi (M=Zn) type coupling reactions between the compounds of general formula **IX** and **III'.** 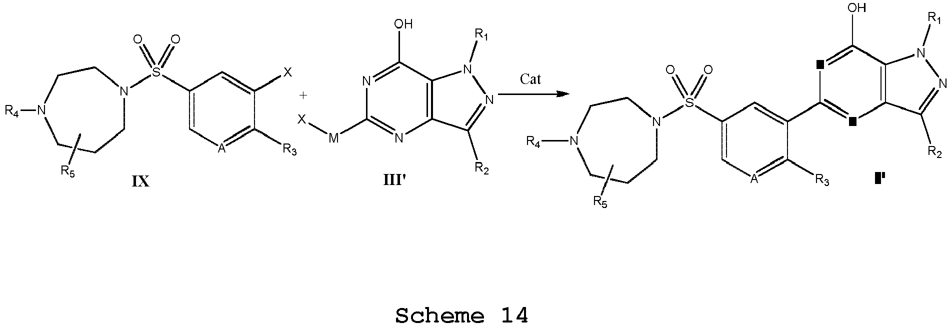
Finally, an iminol-amide tautomeric equilibrium is established between the compounds of general formula **I'-I,** as described in **Scheme 11** of the previous methods.

The stages of the method modalities described above, according to the present invention, can be performed under favored reaction conditions in an appropriate solution system, where an inert organic solvent or an aqueous medium is used, or a mixture of organic solvents with an aqueous medium is used, in the presence of an organic or inorganic base, at ambient temperature or a low temperature, in an oxidative or inert atmosphere, at atmospheric pressure or a low pressure.

Among the preferred reaction conditions for coupling of the compounds of general formula **VI'** and **VII,** according to the present invention, are the molar ratios of said compounds, which can be from 1:5 to 1:1, respectively, depending on the substituent R₄. It is also preferable to use an organic solvent in the presence of an organic base at ambient temperature, in an inert atmosphere and at atmospheric pressure.

The organic solvent is selected from among a group of aromatic, aliphatic, alicyclic or chlorinated solvents and is stable under acidic and alkaline conditions, and in addition is not an inhibitor of the reaction. The solvents preferably used are ethanol, chloroform, or dichloromethane. The amount of solvent used in relation to the compound of general formula **VI',** is preferably in the range 1:0.5 to 1:60 (p/v), respectively, with the most preferred ratio being 1:1 to 1:30. Specifically, the ratio 1:1 to 1:20 is preferred.

The inorganic bases preferably used are NaOH and KOH. The stoichiometric ratio used for the inorganic base is from 0.5:1 to 1.2:1, with respect to the compound of general formula **VI',** preferably the ratio with which the best yields are obtained are 0.8:1 and 1:1, respectively.

The organic bases preferably used in the coupling process between the compounds of general formula **VI'** and **VII** are TEA (triethylamine), DIPEA (*N*,*N'*-diisopropylethylamine), NMM (*N*-methylmorfoline), DABCO (1,4-diazabicyclo[2.2.2]octane) and dimethylphenylamine. The stoichiometric ratio used for the organic base and the compound of general formula **VI'** is from 1:1 to 5:1; with the preferred ratio being 1.5:1.

The reaction stages of the modalities described above, in accordance with the present invention, can preferably be performed at temperatures from -5 to 25°C, the most preferred temperatures being from 0 to 20°C. Specifically, it is preferred that the reaction stages be carried out at temperatures from 3 to 13°C.

The stages of the methods described above, according to the present invention, can be performed at atmospheric pressure, reduced pressure or positive pressure; preferably, synthesis conditions are performed at atmospheric pressure.

Another option for performing the coupling reaction between the compound of general formula **VI'** with the compound of general formula **VII** relates to the reaction conditions, which are characterized by the absence of a solvent, the presence of a suitable organic solvent, ambient temperature and atmospheric pressure.

In an additional modality, the most preferred compounds of the present invention, namely the compounds **2, 3, 4** and **7,** can be prepared by acylation and alkylation processes from a preferred compound of the present invention of general formula **Ia,** which is obtained by any of the above-described methods.

### Acylation method of the compound of general formula Ia.

This method is characterized by making react the compound of general formula **Ia** (compound **I** where R₄ is a proton), 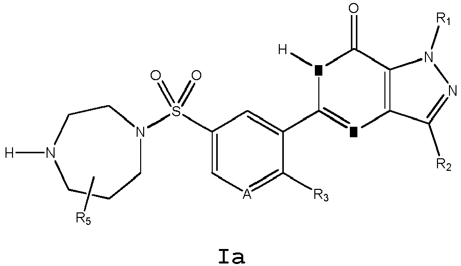 wherein:
A, R₁, R₂, R₃ and R₅ represent functional groups that have been defined previously for the compound of general formula **I,**
with an acid chloride, such as with acetyl chloride, in the presence of a base such as pyridine or triethylamine, to obtain the compound of general formula **Ib,** 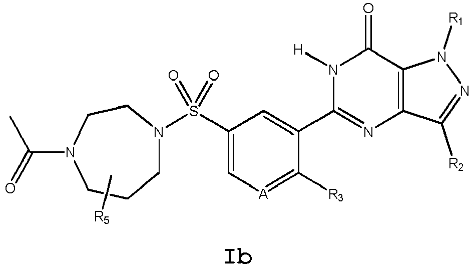
wherein:
A, R₁, R₂, R₃ and R₅ represent functional groups that have been defined for the compound of general formula **I.**

The compound of general formula **Ib** can also be prepared by a reaction with acetic acid in the presence of a coupling agent, such as carbodiimide reagents, for example, DCC (1,3-dicyclohexylcarbodiimide), DIC *(N,N'-*diisopropylcorbodiimide), EDC (N-(3-dimethylaminopropyl)-*N*'-ethylcarbodiimide), among others.

### Alkylation method of the compound of general formula Ia.

This method is characterized by making react the compound of general formula **Ia** (compound **I** where R₄ is a proton), 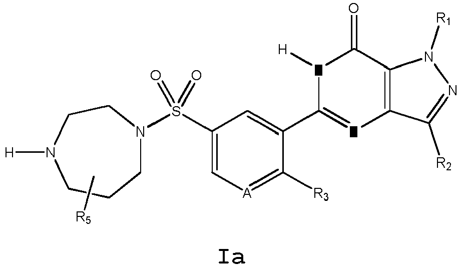 wherein:
A, R₁, R₂, R₃ and R₅ represent functional groups that have been defined for the compound of general formula **I,**
with alkyl halides of general formula **X,** 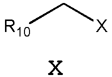
wherein:
R₁₀ represents a proton, or an alkyl with a linear or branched chain of up to seven carbon atoms, optionally substituted with hydroxyl groups or carboxylic acid at the end of the chain, and X represents a halogen atom, such as fluorine, chlorine, bromine or iodine,
to obtain the compound of general formula **Ic,** 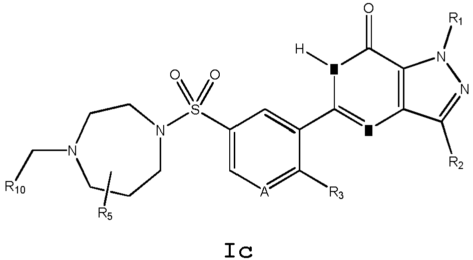
wherein:
A, R₁, R₂, R₃ and R₅ represent functional groups that have been defined previously for the compound of general formula **I,** and
R₁₀ preferably represents a proton or a linear or branched alkyl with up to seven carbon atoms, as in compound **3,**
or represents preferably a linear or branched alkyl chain of up to seven carbon atoms with a hydroxyl group attached at the end of the chain, as in compound **4,**
or represents preferably a linear or branched alkyl chain of up to seven carbon atoms with a carboxylic acid attached at the end of the chain, as in compound **7.**

### Clinical Use.

In an additional aspect of the present invention, it has been found that the compound of general formula **I,** as well as its physiologically acceptable salts, present a satisfactory and unexpected pharmacological activity in animals, specifically in mammals, among which are included human beings.

Specifically, it has been found that the compound of general formula **I,** as well as its physiologically acceptable salts, show an effective and selective inhibitory activity against the phosphodiesterases responsible for metabolizing cyclic guanosine-3',5'-monophosphate (cGMP), such as type 5 phosphodiesterase (PDE-5), which causes an increase in the concentration of circulating cGMP, with beneficial health effects such as the relaxation of the smooth muscles present in the urogenital system, among others.

Therefore, the compound of general formula **I,** according to the present invention, as well as its physiologically acceptable salts, are suitable for the prophylaxis and/or treatment of diseases related to metabolisms regulated by cGMP, preferably those in which it is advantageous to the health to increase the concentration of cGMP, as well as the inhibition of PDE-5.

According to an additional aspect of the present invention, the use of the compound of general formula **I** or of its physiologically acceptable salts is provided, in the preparation of a medicament useful for the prophylaxis and/or treatment of diseases related to metabolisms regulated by cGMP, preferably those in which it is advantageous to the health to increase the concentration of cGMP, as well as the inhibition of PDE-5.

In this manner, the use is enabled of the compound of general formula **I** or of its physiologically acceptable salts in the preparation of a medicament useful in the prophylaxis and/or treatment of erectile dysfunction.

A person skilled in the art will appreciate that certain protected derivates of the compound of general formula **I,** which can be prepared before a final unprotected stage, may not have pharmacologic activity as such, but in some cases can be administered by pharmaceutically acceptable paths and be subsequently metabolized in the body to form the compounds of the invention, which are pharmacologically active. Therefore, such derivates can be described as 'prodrugs'. Additionally, certain compounds of general formula **I** can act as prodrugs of other compounds of general formula **I.**

All the protected derivates and prodrugs of compounds of general formula **I** are included in the scope of the present invention.

Moreover, as an additional aspect of the present invention, a method for treating or preventing diseases related to metabolisms regulated by cGMP is provided, preferably those for which it is beneficial to the health to increase the concentration of cGMP, as well as inhibit PDE-5 in an animal, specifically in mammals, among which human beings are included, comprising the administration of a therapeutically effective amount of the compound of general formula **I** or of its physiologically acceptable salts to a mammal requiring such treatment.

### Pharmaceutical Compositions.

According to an additional aspect of the present invention, the compound of general formula **I** or its physiologically acceptable salts, can be administered by oral, buccal, sublingual, parenteral, for example, intramuscular, intravenous, intradermic or subcutaneous, topical, transdermal, tracheal, bronchial, nasal, pulmonary, rectal or other suitable administration methods.

According to the present invention, the compound of general formula **I** or its physiologically acceptable salts can be formulated as pharmaceutical compositions for human use, comprising any of the active compounds or their physiologically acceptable salts, mixed with at least one coadjuvant agent, diluent agent and/or carrier that are pharmaceutically acceptable, chemically inert, and non-toxic, selected according to the designated administration method and dosage form appropriate for its administration.

The compound of general formula **I** or its physiologically acceptable salts can be contained in pharmaceutically acceptable dosage forms, which include but are not limited to: tablets, chewable tablets, capsules, hard and soft gelatin capsules (including microcapsules), pills, solution, parenteral solutions, lotions (creams and gels), suppositories, pharmaceutical packets and powder for reconstitution or addition to foods, among other dosage forms comprised in this invention.

Using common pharmaceutical processes known in the art, the compound of general formula **I** or its physiologically acceptable salts can be made in dosage forms suitable for administration, mixing them with auxiliary substances such as: commonly used liquid or solid excipients, ingredients in powder form, pharmaceutically acceptable liquids or filler solids, diluents, agglutinants, solvents, emulsion agents, lubricants, disintegrating agents, sliding agents, flavoring agents, colorants, pigments, polymers, sweeteners, plasticisers, absorption enhancers, penetration enhancers, surfactants, cosurfactants, specialized oils and/or buffer solutions, which provide the active compounds or their physiologically acceptable salts with physical, chemical and/or biological stability.

Among the auxiliary substances used in the manufacture of the dosage forms containing the compound of general formula **I** or its physiologically acceptable salts include but are not limited to: magnesium carbonate, titanium dioxide, lactose, saccharose, sorbitol, manitol and other sugars, talcum, lacto-protein, gelatine, starch, amylopectin, cellulose and their derivates, animal and vegetable oils such as fish liver oil, sunflower oil or sesame oil, polyethyleneglycol and solvents such as for example sterile water and mono or polyhydroxylic alcohols such as glycerol, as well as disintegrating agents and lubricating agents such as magnesium stereate, calcium stereate, sodium fumarate stearate and polyethylene glycol waxes.

Other examples of excipients include: calcium phosphates, such as dibasic calcium phosphate, anhydrous dibasic calcium phosphate, tribasic calcium phosphate, etc.; microcrystalline cellulose, starch, pregelatinised starch, sodium glycolate starch; manitol, sorbitol, povidone; ethylcellulose, cyclodextrins, lactose, kaolin, silicic acid, lubricants, such as magnesium stereate, calcium stearate, mineral oil, glycerin, sodium lauryl sulfate, polyethyleneglycol and/or talcum.

The compound of general formula **I** or their physiologically acceptable salts can also be mixed with other drugs or coadjuvants which act by stimulating the production of nitric oxide, such as calcium dobesilate, coenzyme Q, L-carnitine and/or L-arginine and their structural analogues, before being combined with the non-active ingredients to form the final pharmaceutical compositions.

Soft gelatine capsules can be prepared by mixing the compound of general formula **I** or its physiological acceptable salts with vegetable oils, fats or other similar vehicles suitable for its formulation. Hard gelatine capsules may contain granules of the compound of general formula **I** or its physiologically acceptable salts. Hard gelatine capsule can also contain the compound of general formula **I** or its physiologically acceptable salts, as well as solid ingredients in powder form, such as lactose, saccharose, sorbitol, manitol, potato starch, corn starch, amylopectin, derivates of cellulose or gelatine, among others.

For rectal administration, the compound of general formula **I** or its physiologically acceptable salts, can be formulated in the form of suppositories that may contain a mixture of the active compounds or their physiologically acceptable salts with a base of neutral grease. Another form of rectal administration can be a rectal gelatin capsule that may contain a mixture of the active compounds or their physiologically acceptable salts with a vegetable oil, a paraffin or another similar vehicle suitable for its formulation.

For liquid oral administration, the compound of general formula **I** or its physiologically acceptable salts, can be formulated in the form of syrups, elixirs, concentrated drops or suspensions, for example, solutions or suspensions that may contain a mixture of the active compounds or their physiologically acceptable salts, a pharmaceutically acceptable vehicle, such as sugar or derivates, and a mixture of ethanol, water, glycerol, propyleneglycol and/or polyethyleneglycol, among others. In addition, the liquid dosage forms containing the compound of general formula **I** or its physiologically acceptable salts, if preferred, may contain colorants, flavorisers, preservatives, saccharine, carboxymethylcellulose or other thickening agents. Moreover, the liquid dosage forms can be prepared by reconstituting pharmaceutical compositions in dry powder with a suitable solvent before their use.

For parenteral administration, the compound of general formula **I** or its physiologically acceptable salts can be formulated in the form of solutions. These solutions may contain stabilizing ingredients, preservatives and/or buffer ingredients. The solutions for parenteral administration containing the compound of general formula **I** or their physiologically acceptable salts can also be prepared by reconstituting a dry pharmaceutical composition with a suitable solvent before their use.

For solid oral administration, the mixtures containing the compound of general formula **I** or its physiologically acceptable salts, in addition to being pharmaceutically acceptable excipients, may be in the form of microgranules, tablets, capsules or pills.

For topical administration, the compound of general formula **I** or its physiologically acceptable salts, can be formulated in the form of lotions, creams or gels that may the active compounds or their physiologically acceptable salts suspended or dissolved, mixed with one or more pharmaceutically acceptable excipients, such as propyleneglycol, polyethyleneglycol, polyoxypropylene, polyoxyethylene, sorbitan monostereate, polysorbate 60, mineral oil, liquid petrolate, liquid paraffin, waxes of emulsioning ethyl esters, benzyl alcohol, cetearyl alcohol, water, among others.

For transdermal administration, the compound of general formula **I** or its physiologically acceptable salts, can be formulated in the form of patches that may contain a mixture of the active compounds or their physiologically acceptable salts mixed with one or more pharmaceutically acceptable excipients suitable for their application.

The pharmaceutical compositions according to the present invention may contain pharmaceutically acceptable polymers, permeable and insoluble in water, to control their release profile, so that it is possible to obtain dosage forms with modified release (immediate, delayed or controlled). These polymers can be used to cover dosage forms such as tablets, microgranules, capsules or pills, or be mixed with the remaining excipients included in any other dosage form mentioned in the present invention.

The solid oral administration forms such as tablets, microgranules, capsules or pills, containing the compound of general formula **I** or its physiologically acceptable salts, according to the present invention, can be of immediate or modified release.

A preferred dosage form according to the present invention can be in the form of tablets, which can comprise the compound of general formula **I** or its physiologically acceptable salts and excipients, such as lactose, microcrystalline cellulose, polyethyleneglycol 6000, magnesium stearate and sodium glycolate starch, among others.

Said tablets can have modified release and comprise the compound of general formula **I** or its physiologically acceptable salts and excipients, such as manitol, microcrystalline cellulose, ethylcellulose, povidone, sodium glycolate starch, talcum, among others.

The tablets according to the present invention can be prepared mixing the compound of the general formula **I** or its physiologically acceptable salts with microcrystalline cellulose, lactose or manitol in powder form, followed by adding a solution of polyethylene glycol or a solution of ethylcellulose and povidone to form a granulated mixture. This granulate is dried and mixed with sodium glycolate starch and magnesium stereate or talcum; the tablets are then made using a system of rotary punches.

In another preferred form of preparation according to the present invention, the tablets can be prepared by mixing the compound of general formula **I** or its physiologically acceptable salts with excipients, such as starch, microcrystalline cellulose, lactose, and magnesium stearate; these ingredients are then compressed to tablet form using a technique known as direct compression.

The taste of the tablets, according to the present invention, can be masked by coating them with a taste masking agent, such as copolymer or methylacrylic acid, methylcellulose or methylhydroxypropylcellulose; preferably, the tablets of the invention are coated with methylhydroxypropylcellulose.

Another preferred dosage form according to the present invention can be in the form of capsules, which can comprise a mixture of the compound of general formula I or its physiologically acceptable salts with excipients, such as lactose, microcrystalline cellulose, polyethyleneglycol, talcum, among others.

A preferred dosage form according to the present invention can also be in the form of pharmaceutical packets, which can be prepared by mixing the compound of general formula I or its physiologically acceptable salts with excipients, such as lactose, microcrystalline cellulose dioxide, silicone, talcum, sodium glycolate starch, starch, among others.

In another preferred dosage form according to the present invention, a mixture of powders is provided to be reconstituted in the form of a syrup, which can comprise the compound of general formula I or its physiologically acceptable salts, an artificial sweetener, preferably sucralose, microcrystalline cellulose and a flavoring agent, as well as any combination of excipients, known in the state of the art, suitable for manufacture a syrup by reconstitution, such as colorants, solvents, among others.

In one modality of the present invention, for use in humans, the total daily amount of the compound of general formula **I** or its physiologically acceptable salts that can be usually administered by the various dosage forms described above, is in the range of 10 to 550 mg of free base, which can be administered in a single dose or in more doses distributed over a day, as required.

Thus, for example, for oral administration doses can be administered of 10 to 550 mg, preferably 25 to 300 mg. In parenteral administration, it is convenient to administer doses of 10 to 20 mg.

In any case, the physician will prescribe the effective dose as deemed appropriate, if applicable modifying the amounts indicated above, as these will depend on the body mass and age of the patient, as well as his/her response to the medicament, among other factors. Therefore, there can be specific and individual circumstances in each patient that make it beneficial or sufficient to administer lower amounts than the minimum amounts described above, while in other cases it may be advantageous to increase said maximum amounts. Consequently, such ranges are considered to fall within the scope of the present invention.

### Examples of the synthesis of the compound of general formula I.

The synthesis of the compound of general formula I, according to the present invention, and of the intermediaries used for this purpose, is illustrated by the following examples.

### Example 1.

### 5-(5-(1,4-diazepane-1-ylsulfonyl)-2-ethoxyphenyl)-1-methyl-3-propyl-1H-pyrazolo[4,3-d]pyrimidin-7(6H)-one (Compound 1) .

A mixture, at ambient temperature and in a nitrogen atmosphere, of 4-ethoxy-3-(1-methyl-7-oxo-3-propyl-6,7-dihydro-1*H*-pyrazolo[4,3-*d*]pyrimidin-5-yl)benzene-1-sulfonyl chloride (1.0 g, 2.4 mmol) in ethanol (10 mL)is slowly added to a mixture of 1,4-diazepane (0.25 g, 2.4 mmol) and triethylamine (0.37 g, 3.64 mmol) in ethanol (10 mL). After one hour, eliminate the solvent, dissolve the product in dichloromethane and wash with brine. Dry the organic phase and eliminate the solvent to obtain compound **1** with a 36.25% yield.

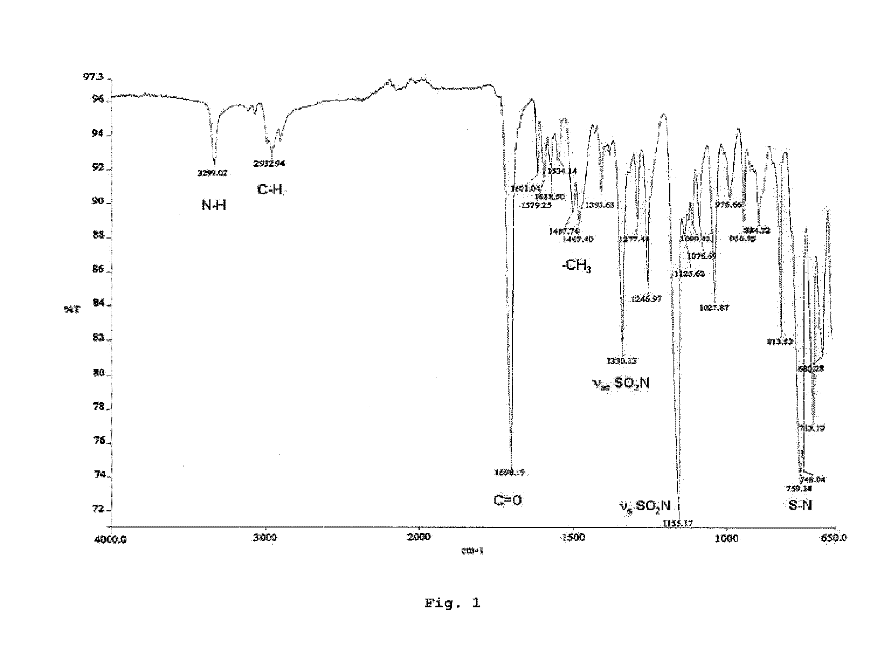

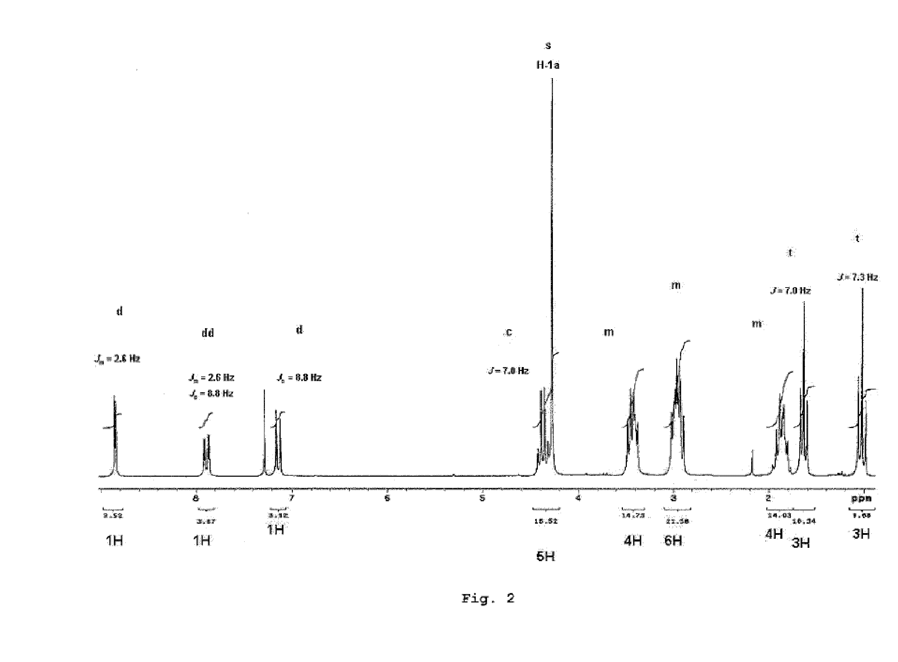

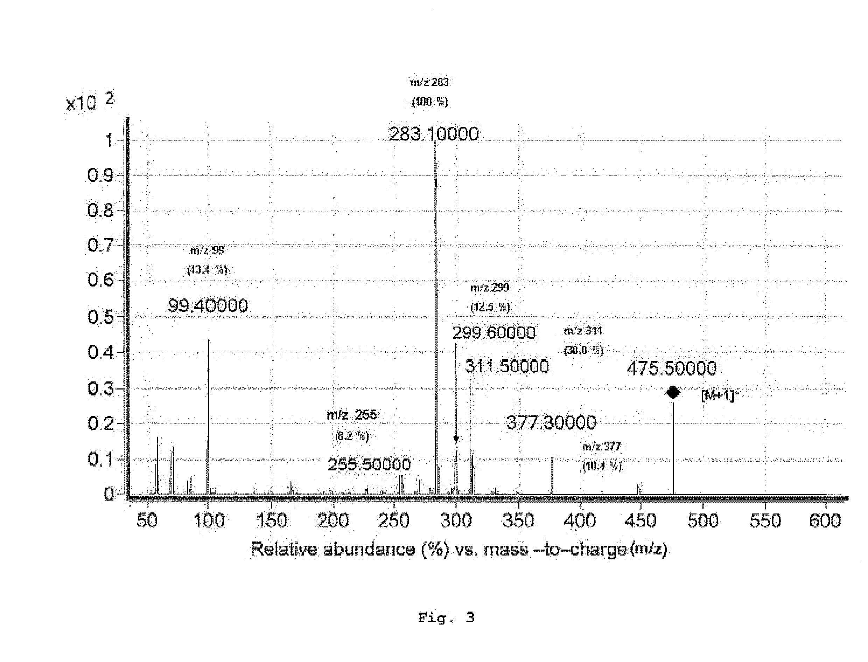

The product was analyzed giving the following results: white solid; m.p. 156-159°C. IR νₘₐₓ (cm⁻¹): 3299, 2932, 1698, 1601, 1534, 1487, 1467, 1330, 1246, 1155, 1027, 813, 759 **(****Figure 1****)**. RMN ¹H (200 MHz, CDCl₃) δ (ppm): 1.02 (t, 3H, *J*= 7.0 Hz); 1.65 (t, 3H, *J*= 7.0 Hz); 1.86 (m, 4H); 2.93 (m, 6H); 3.45 (m, 4H); 4.25 (s, 3H); 4.35 (c, 2H, *J=* 7.0 Hz); 7.14 (d, 1H, *Jₒ*= 8.8 Hz); 7.88 (dd, 1H, *Jₘ*= 2.6 Hz, *Jₒ*= 8.8 Hz); 8.85 (d, 1H, *Jₘ*= 2.6 Hz); 10.91 (a, 1H), (**Figure 2**)**.** ESI/EM: [M+1]⁺ 475 m/z (**Figure 3**)**.**

### Examples 2 to 6.

### Optimisation for obtaining the compound 5-(5-(1,4-diazepane-1-ylsulfonyl)-2-ethoxyphenyl)-1-methyl-3-propyl-1H-pyrazolo[4,3-d]pyrimidin-7(6H)-one (Compound 1).

Examples 2 to 6 were obtained under the experimental conditions described for example 1, using different stoichiometric ratios between the substrates, as well as the experimental modifications specified in **Table 2.**

**Table 2. Conditions for optimizing the compound 1.**

| Example | Temperature (°C) | Molar ratio | Yield (%) | Similarity factor¹ |
|---|---|---|---|---|
| 2 | T.A. | 1:4 | 85.5 | 0.98 |
| 3 | T.A. | 1:5 | 94.5 | 0.98 |
| 4 | 0-10 | 1:1 | 39.5 | 0.96 |
| 5 | 0-10 | 1:4 | 89.0 | 0.97 |
| 6 | 0-10 | 1:5 | 98.0 | 0.98 |

| | | | | |
|---|---|---|---|---|
| ¹ Comparison of the infrared absorption spectrum. | | | | |

### Example 7.

### Preparation of derivates of 1,4-diazepanes. tert-Butyl 1,4-diazepane-1-carboxylate.

To a solution of di-tert-butyldicarbonate (2.201 g, 9.780 mmol) in acetic acid (9.8 mL), a solution of 1,4-diazepane (1.0 g, 9.780 mmol) in acetic acid (9.8 mL)at ambient temperature is added. After 24 hours, add water and adjust the pH to 10 with 10% NaOH. Extract the aqueous phase with dichloromethane (2 x 15 mL), dry the organic phase and eliminate the solvent, to obtain the compound tert-butyl 1,4-diazepane-1-carboxylate with 90.0% yield.

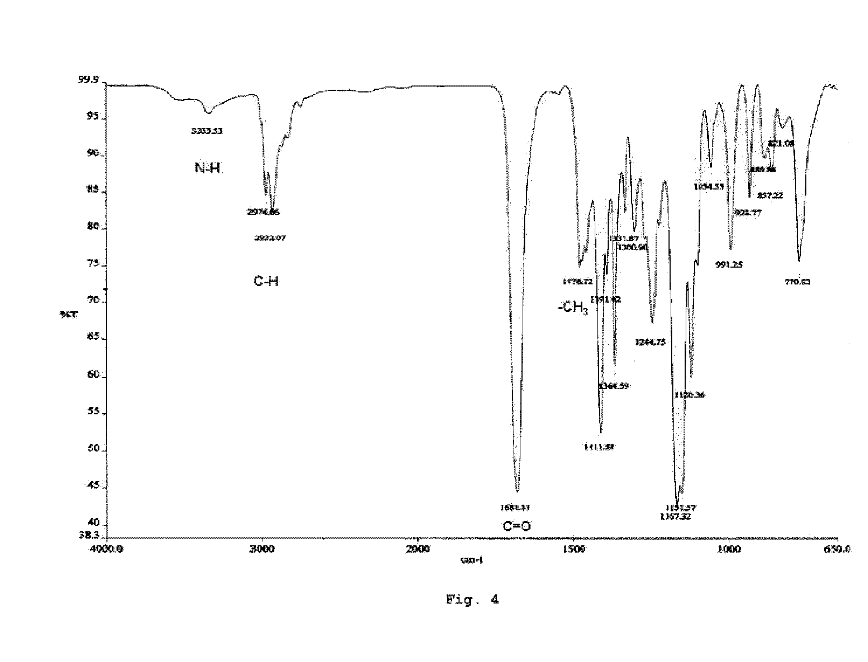

A liquid is obtained that was analyzed, obtaining the following data: IR νₘₐₓ (cm⁻¹): 2932, 2514, 1681, 1477, 1411, 1166, 991, 769 (**Figure 4**)**.**

### Examples 8 to 11.

### Preparation of the compounds of general formula 2-5.

Examples 8 to 11 were performed under the experimental conditions described in example 1, changing only the stoichiometric ratio to 1:1.1 for the chlorosulfonyl derivate and the suitable derivates of 1,4-diazepane, respectively (**Table 3**)**.**

**Table 3. Preparation of the compounds 2-5.**

| Example | Compound | R₄ | Yield (%) | Melting point (°C) |
|---|---|---|---|---|
| 8 | **2** | COMe | 93.0 | 150-152 |
| 9 | **3** | Me | 92.0 | 136-138 |
| 10 | **4** | HO(CH₂)₃ | 92.0 | 122-124 |
| 11 | **5** | Boc | 90.0 | 146-148 |

All compounds of examples 8 to 11 were characterized by their melting point and their IR, RMN ¹H, EM spectroscopic data.

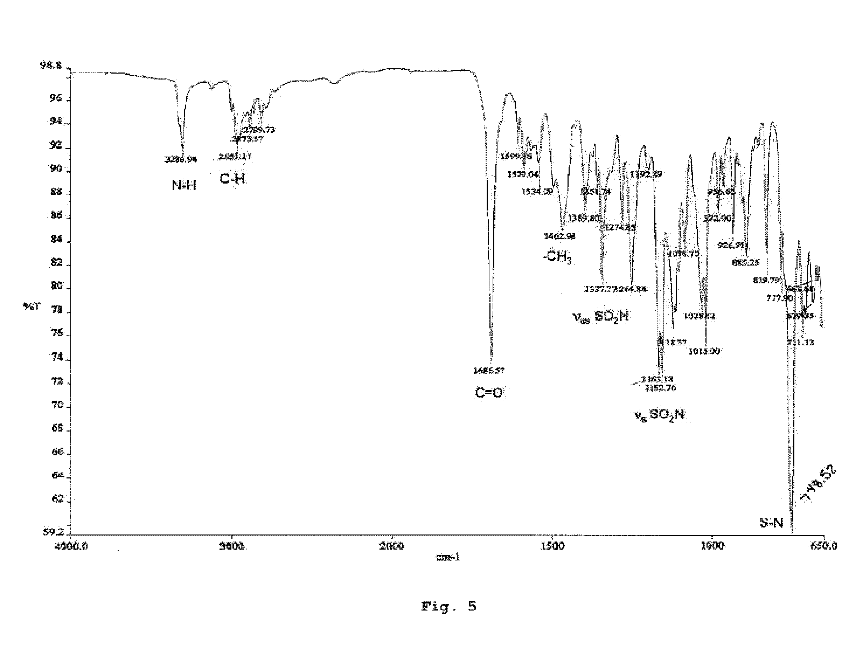

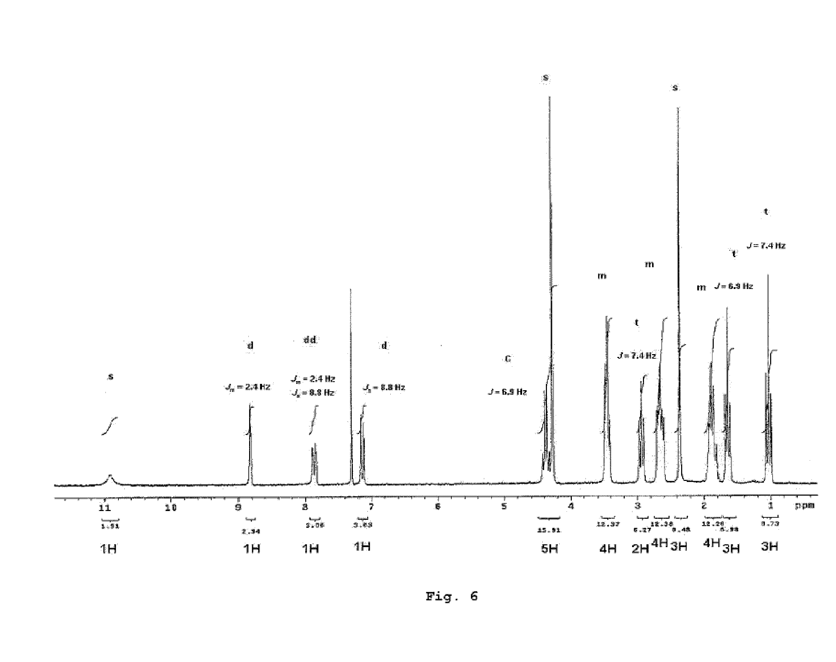

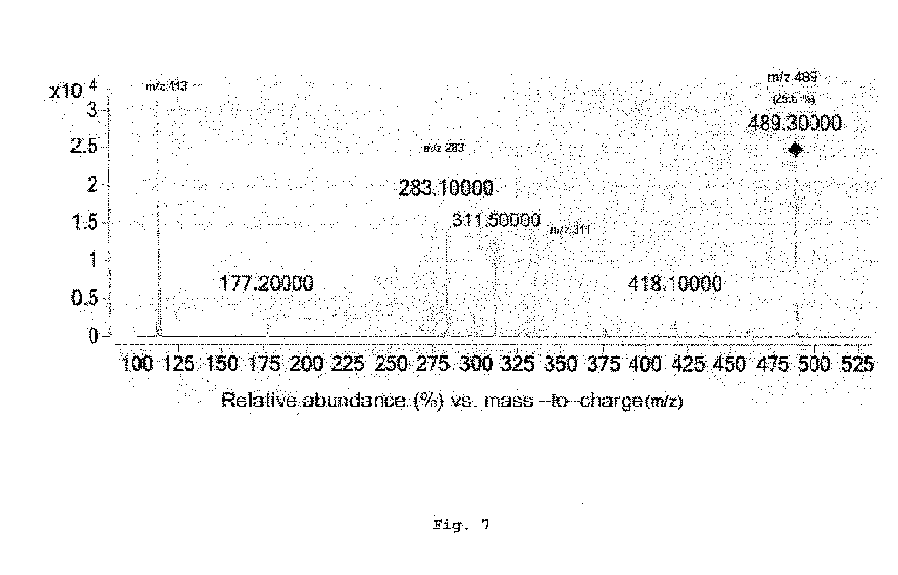

For example, for compound **3** a white solid was obtained; m.p. 136-138°C. IR νₘₐₓ (cm⁻¹): 3287, 2951, 1687, 1463, 1390, 1338, 1245, 1163, 1153, 1028, 1015, 885, 820, 749 (**Figure 5**). RMN ¹H (200 MHz, CDCl₃) δ (ppm): 1.03 (t, 3H, *J=* 7.4 Hz); 1.64 (t, 3H, *J=* 6.9 Hz); 1.86 (m, 4H); 2.35 (s, 3H); 2.65 (m, 4H); 2.93 (t, 2H, *J=* 7.4 Hz); 3.44 (m, 4H); 4.26 (s, 3H); 4.37 (c, 2H, *J*= 6.9 Hz); 7.13 (d, 1H, *Jₒ*= 8.8 Hz); 7.86 (dd, 1H, *Jₘ*= 2.4 Hz, *Jₒ*= 8.8 Hz); 8.82 (d, 1H, *Jₘ*= 2.4 Hz); 10.91 (s, a, 1H) (**Figure 6**)**.** ESI/EM: [M+1]⁺ 489 m/z (**Figure 7**).

### Examples 12 to 15.

### Optimisation for obtaining compound 3.

Examples 12 to 15 were performed under the experimental conditions described in example 9, with the modifications described in **Table 4.**

**Table 4. Conditions for optimizing compound 3.**

| Example | Molar ratio of substrates | No. of equivalents of Et₃N | P/V ratio Substrate-Solvent. | Solvent | Yield (%) |
|---|---|---|---|---|---|
| 12 | 1:1.20 | 3.0 | 1:40 | EtOH | 97.0 |
| 13 | 1:1.05 | 1.5 | 1:20 | EtOH | 97.5 |
| 14 | 1:1.05 | 1.5 | 1:20 | DCM | 95.0 |
| 15 | 1:1.05 | 1.5 | 1:1 | DCM | 95.0 |

The products were compared with the melting point and IR spectrum of the product in example 9, all of which were equal or more than 0.95 similar.

### Example 16.

### Preparation of the compound 3 in the absence of solvent.

To a mixture of 4-ethoxy-3-(1-methyl-7-oxo-3-propyl-6,7-dihydro-1*H*-pyrazol[4,3-d]pyrimidin-5-yl)benzene-1-sulfonyl (0.5 g, 1.21 mmol) and triethylamine (0.25 mL, 1.82 mmol) chloride, add slowly 1-methyl-1,4-diazepane, (0.16 mL, 0.15 mmol)at ambient temperature is slowly added. After 10 min., dissolve the product in dichloromethane and wash with brine. Dry the organic phase and eliminate the solvent to obtain compound 3 with a 85.5% yield.

### Alternative methods for manufacture compounds 2, 3 and 5.

### Example 17.

### Acetylation of compound 1 to obtain 5-(5-(4-acetyl-1,4-diazepane-1-ylsulfonyl)-2-ethoxyphenyl)-1-methyl-3-propyl-1H-pyrazole[4,3-d]pyrimidin-7(6H)-one (Compound 2).

A solution and in a nitrogen atmosphere of DCC (0.231 g, 1.111 mmol) and DMAP (0.026 g, 0.212 mmol) in anhydrous dichloromethane (5 mL) to a suspension 5-(5-(1,4-diazepane-1-ylsulfonyl)-2-ethoxyphenyl)-1-methyl-3-propyl-1*H-*pyrazolo[4,3-*d*]pyrimidin-7(6*H*)-one (Compound 1, 0.5 g, 1.058 mmol) and acetic acid (0.064 g, 1.058 mmol) in anhydrous dichloromethane (5 mL)at ambient temperature is slowly added. After 12 hours, filter the suspension and wash the solution with 1.0 N HCl and brine. Dry the organic phase and eliminate the solvent, recrystallising the crude product of CHCl₃:*n-*hexane, obtaining compound 2 with a yield of 80.0%.

Compound 2 was compared to the product obtained in example 8 in its melting point (150-152°C) and infrared absorption spectrum, with a similarity factor of 0.95.

### Example 18.

### Reductive alkylation of compound 1 to obtain 5-(2-ethoxy-5-(4-methyl-1,4-diazepan-1-ylsulfonyl)phenyl)-1-methyl-3-propyl-1H-pyrazolo[4,3-d]pyrimidin-7(6H)-one (Compound 3).

To a mixture of 37% formaldehyde (1.74 g, 1.71 mL, 21.15 mmol) and formic acid (0.974 g, 0.798 mL, 21.15 mmol), add slowly and constantly, with constant stirring 5-(5-(1,4-diazepan-1-ylsulfonyl)-2-ethoxyphenyl)-1-methyl-3-propyl-1*H-*pyrazolo[4,3-*d*]pyrimidin-7(6*H*)-one (Compound **1,** 0.5 g, 1.058 mmol)).

Stir the mixture at a temperature of 50-55°C for 12.0 h. After cooling the mixture with excess water, extract the product with dichloromethane, dry the organic phase and eliminate the solvent, obtaining compound **3** with a yield of 87%.

Compound **3** was compared to the product obtained in example 9 in its melting point (134-136°C) and infrared absorption spectrum, with a similarity factor of 0.96.

### Example 19.

### Reductive alkylation of compound 1 with zinc to obtain 5-(2-ethoxy-5-(4-methyl-1,4-diazepan-1-ylsulfonyl)phenyl)-1-methyl-3-propyl-1H-pyrazolo[4,3-d]pyrimidin-7(6H)-one (Compound 3).

To a solution at ambient temperature of 5-(5-(1,4-diazepan-1-ylsulfonyl)-2-ethoxyphenyl)-1-methyl-3-propyl-1*H-*pyrazolo[4,3-*d*]pyrimidin-7(6*H*)-one (Compound **1**, 0.5 g, 1.058 mmol) and acetic acid (0.255 g, 0.243 mL, 4.231 mmol) in water (2 mL). Subsequently, formaldehyde (0.131 g, 0.128 mL, 1.587 mmol) and zinc shavings (0.138 g, 2.116 mmol)is added, heat the solution to 30°C, and after 8 hours add excess water and extract the product with dichloromethane; dry the organic phase, eliminate the solvent and purify the product with a chromatographic column in CHCl₃:MeOH (95:5), obtaining compound 3 with a yield of 70.7%.

Compound 3 was compared to the product obtained in example 9 in its melting point (138-140°C) and infrared absorption spectrum, with a similarity factor of 0.98.

### Example 20.

### Boc protection of compound 1 to obtain tert-butyl 4-(4-ethoxy-3-(1-methyl-7-oxo-3-propyl-6,7-dihydro-1H-pyrazolo[4,3-d]pyrimidin-5-yl)phenylsulfonyl)-1,4-diazepan-1-carboxylate (Compound 5).

To a solution at 0°C of 5-(5-(1,4-diazepan-1-ylsulfonyl)-2-ethoxyphenyl)-1-methyl-3-propyl-1*H-*pyrazolo[4,3-*d*]pyrimidin-7(6*H*)-one (compound 1, 0.5 g, 1.058 mmol) and triethylamine (0.215 g, 0.296 mL, 2.116 mmol) in dichloromethane (5 mL), a solution of di-tert-butyldicarbonate (0.238 g, 1.058 mmol) in dichloromethane (5 mL)is added. After one hour at ambient temperature, wash the solution with brine. Dry the organic phase and eliminate the solvent, recrystallising the crude product of CHCl₃:*n*-hexane, obtaining compound **5** with a yield of 88.0%.

Compound **5** was compared to the product obtained in example 9 in its melting point (146-148°C) and infrared absorption spectrum, with a similarity factor of 0.97.

### Preparation of the physiologically acceptable salts

### Example 21.

### 4-(4-ethoxy-3-(1-methyl-7-oxo-3-propyl-6,7-dihydro-1H-pyrazolo[4,3-d]pyrimidin-5-yl)phenylsulfonyl)-1-methyl-1,4-diazepanium citrate (Citrate of compound 3).

To a mixture with constant stirring and at reflux temperature of 5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one (compound **3,** 5.0 g, 10.23 mmol) and ethanol (32 mL), a solution of citric acid (1.97 g, 10.23 mmol) in ethanol (12 mL)is slowly added. After one hour, allow to reach ambient temperature and filter the precipitated product, obtaining the citrate of compound 3 with a yield of 80.0%.

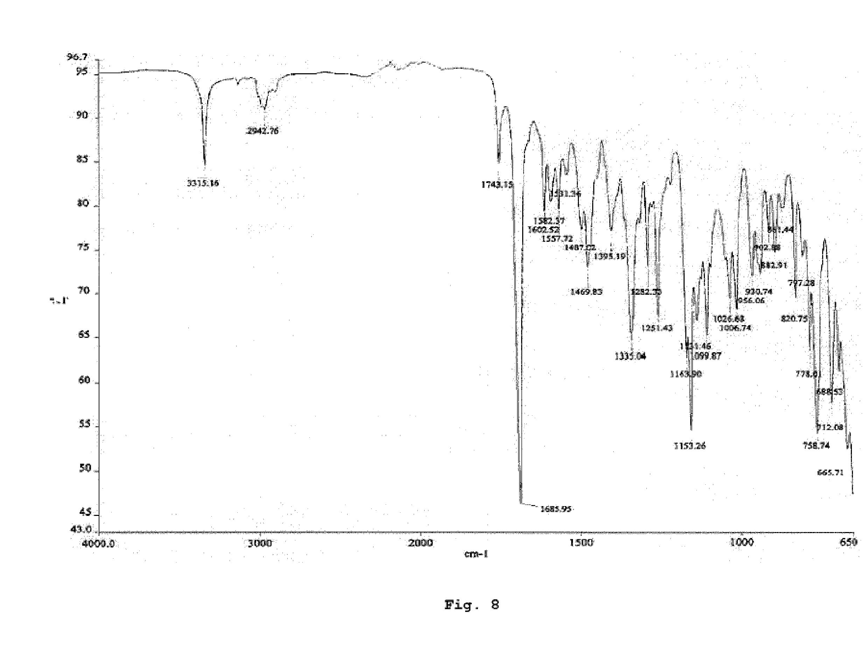

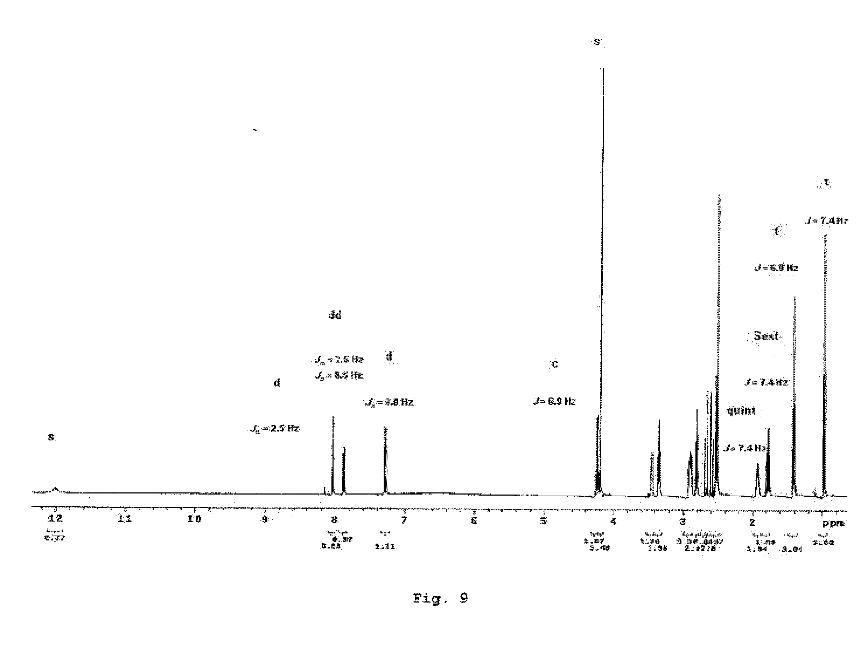

The salt formed was analyzed giving the following results: white solid; m.p. 184-186°C. IR νₘₐₓ (cm⁻¹): 3315, 2943, 1743, 1686, 1470, 1335, 1153, 1007, 821, 759, 666 (**Figure 8**). RMN ¹H (500 MHz, DMSO) δ (ppm): 0.975 (t, 3H, *J=* 7.5 Hz); 1.40 (t, 3H, *J=* 7.0 Hz); 1.78 (sx, 2H, *J=* 7.4 Hz); 1.93 (m, 2H); 2.52 (s, 3H); 2.55 (d, 1H, *J=* 15 Hz); 2.68 (d, 1H, *J=* 15 Hz); 2.81 (t, 2H, *J=* 7.5 Hz); 2.90 (m, 3H); 3.35 (t, 2H, *J=* 6.5 Hz); 3.45 (m, 2H); 4.20 (s, 3H); 4.25 (c, 2H, *J=* 7.0 Hz); 7.28 (d, 1H, *Jₒ*= 9.0 Hz); 7.87 (dd, 1H, *Jₘ*= 2.5 Hz, *Jₒ*= 8.5 Hz); 8.82 (d, 1H, *Jₘ*= 2.5 Hz); 12.0 (s a, 1H) (**Figure 9**).

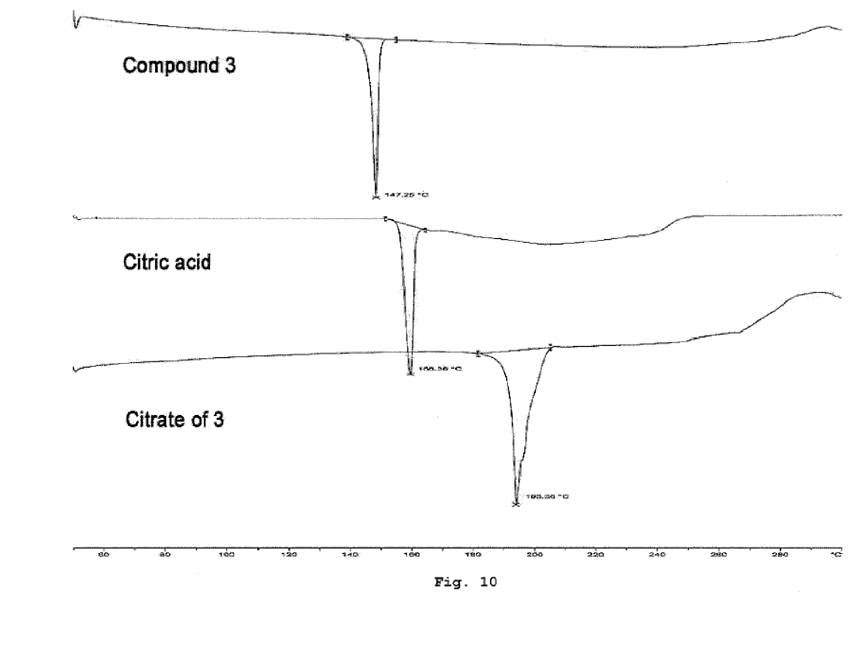

In addition, the citrate salt of compound **3** is characterized by an endothermic peak at 193°C, unlike the endothermic peak observed for base compound **3** which appears at 147°C, as shown in the thermograph of the differential scanning calorimetry analysis (**Figure 10**).

### Example 22.

### Citrate of compound 3 in acetone.

Example 22 was performed under the experimental conditions described in example 21, but using acetone as the solvent. The citrate salt of compound **3** was obtained with a yield of 45.0%.

### Example 23.

### Chlorohydrate of 4-(4-ethoxy-3-(1-methyl-7-oxo-3-propyl-6,7-dihydro-1H-pyrazolo[4,3-d]pyrimidin-5-yl)phenylsulfonyl)-1-methyl-1,4-diazepanium (chlorohydrate of compound 3).

To a mixture with constant stirring and at reflux temperature of 5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one (compound 3, 1 g, 2.05 mmol) with acetone (20 mL), add slowly a 37% HCl solution (0.20 mL, 2.25 mmol) in 2-propanol (3.7 mL). After two hours, add n-heptane (10 mL) and allow to reach ambient temperature. Filter the precipitated product to obtain the chlorohydrate of compound **3** with a yield of 68.29%.

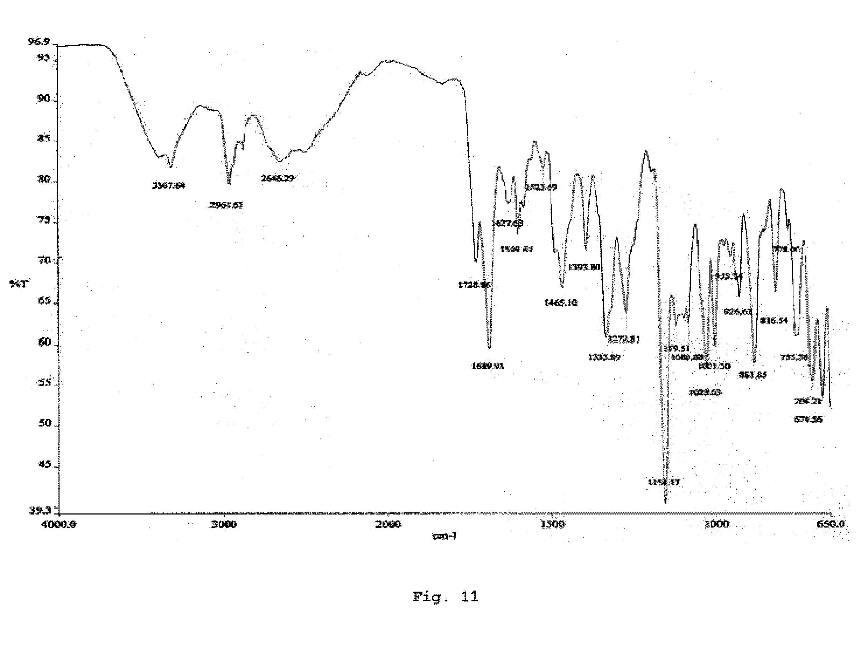

The salt formed was analyzed giving the following results: white solid; m.p. 172-174°C. IR **υ**ₘₐₓ (cm⁻¹): 3308, 2962, 2646, 1729, 1690, 1465, 1334, 1273, 1154, 1028, 817, 755, 675 (**Figure 11**).

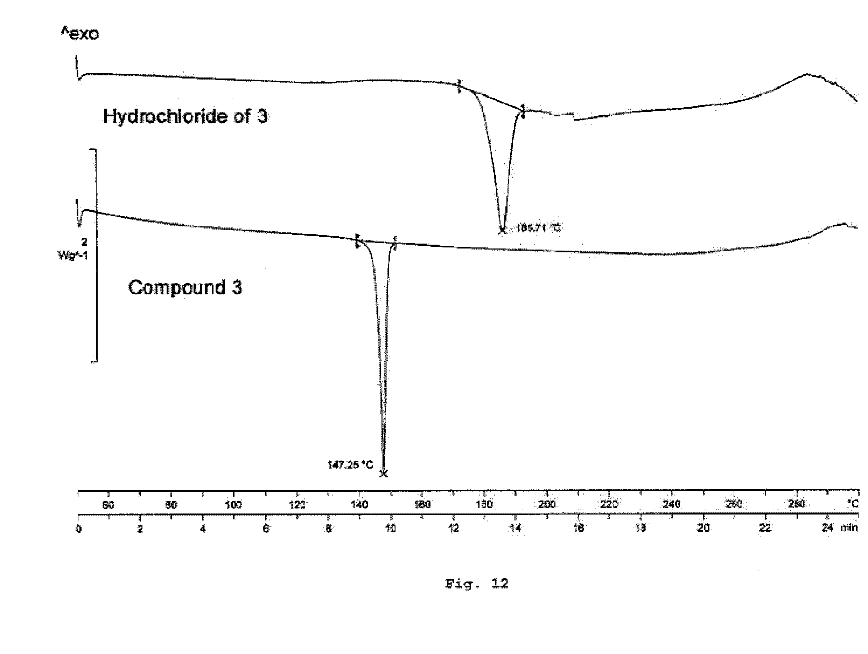

In addition, the chlorohydrate salt of compound **3** is characterized by an endothermic peak at 186°C, unlike the endothermic peak observed for base compound **3,** which appears at 147°C, as shown in the thermograph of the differential scanning calorimetry analysis (**Figure 12**).

### Example 24.

### 4-(4-ethoxy-3-(1-methyl-7-oxo-3-propyl-6,7-dihydro-1H-pyrazolo[4,3-d]pyrimidin-5-yl)phenylsulfonyl)-1-methyl-1,4-diazepanium tartrate (Tartrate of compound 3).

To a mixture with constant stirring and at reflux temperature of 5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one (compound **3,** 1 g, 2.03 mmol) and ethanol (22 mL), add slowly a solution of L-(+)-tartaric acid (0.46 g, 3.04 mmol) in ethanol (25 mL). After one hour, allow to cool to ambient temperature and keep stirring for 14 h. Filter the precipitated, obtaining the tartrate of compound **3** with a yield of 65.0%.

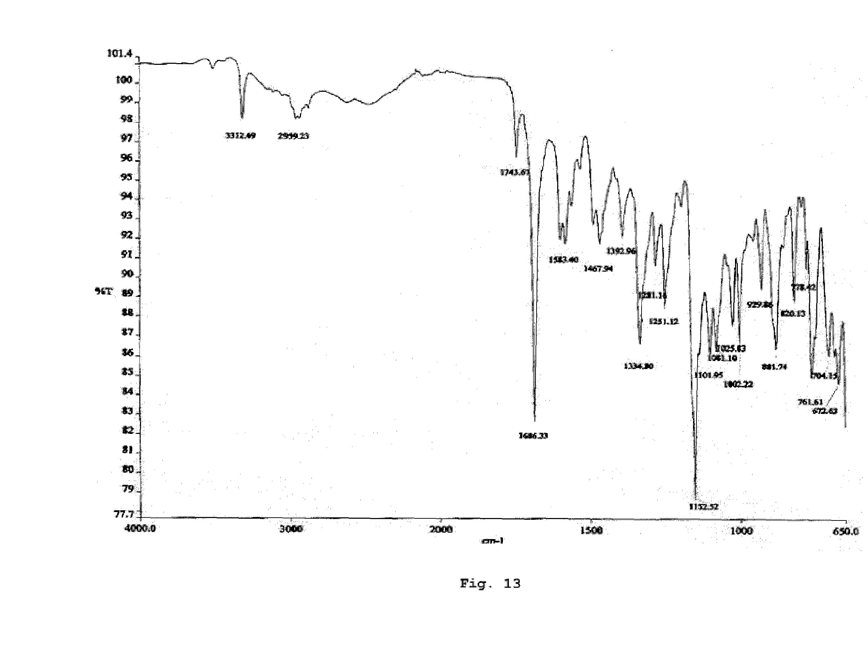

The salt formed was analyzed giving the following results: white solid; m.p. 182-186°C. IR **υ**ₘₐₓ (cm⁻¹): 3312, 2959, 1743, 1686, 1583, 1467, 1392, 1334, 1251, 1152, 1002, 881, 820, 761 (**Figure 13**).

### N-oxidation of compounds of general formula I.

### Example 25.

### Oxidation with hydrogen peroxide of compound 3.

### 4-(4-ethoxy-3-(1-methyl-7-oxo-3-propyl-6,7-dihydro-1H-pyrazolo[4,3-d]pyrimidin-5-yl)phenylsulfonyl)-1-methyl-1,4-diazepane 1-oxide (N-oxide of compound 3).

To a solution at ambient temperature of 5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one (compound **3,** 0.50 g, 1.03 mmol) in dichloromethane (26 mL), add slowly and in small amounts 30% hydrogen peroxide (3.03 g, 2.73 mL, 26.7 mmol). The reaction mixture is maintained at ambient temperature with stirring for 48 h. Concentrate the solution, obtaining a solid with a yield of 85%.

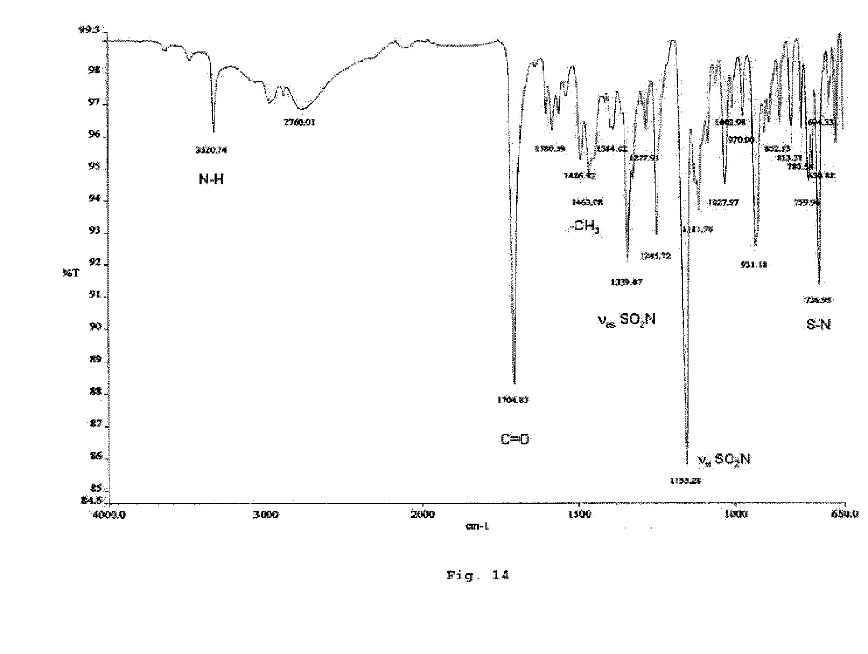

Analyse the product in its melting point (176-180°C) and infrared absorption spectrum, IR νₘₐₓ (cm⁻¹): 3320, 2760, 1704, 1580, 1486, 1463, 1339, 1155, 1027, 931, 726 (**Figure 14**)**.**

### solubility tests.

### Example 26.

### Solubility of the citrate salt of compound 3 in water.

In a test tube, place 100 mg of the citrate salt of compound 3 and add 1 mL of water. Stir the sample for 2 min. and continue in this way until all of the salt has dissolved.

Perform the test in triplicate at ambient temperature. The 100 mg of the citrate salt of compound **3** dissolve in 15 mL of water, so that its solubility is 6.6 mg/mL; thus, this salt is classified as poorly soluble according to the approximate solubilities table for pharmacopeic substances of USP 31.

### Examples 27 to 37.

### Solubility of the citrate salt of compound 3 in organic solvents.

Examples 27 to 37 were performed following the same method as example 26, changing only the solvents with the citrate, chlorohydrate and tartrate salts for the compound of formula **3** (**Table 5**).

**Table 5. Solubility (mg/mL) of the salts of compound 3.**

| | Water | EtOH | CHCl₃ | DMSO |
|---|---|---|---|---|
| Examples | 26 | 27 | 28 | 29 |
| **Citrate** | 6.6 | < 0.6 | 16.0 | 166.0 |
| Examples | 30 | 31 | 32 | 33 |
| **Chlorohydrate** | 166.0 | 33.0 | 125.0 | 250.0 |
| Examples | 34 | 35 | 36 | 37 |
| **Tartrate** | 125.0 | 1.6 | - | 50.0 |

### Biological Assays.

The biological activity of the compounds according to the present invention was determined by the test methods described below.

### Phosphodiesterase 5 (PDE-5) inhibition activity in in vitro tests.

The compounds of the present invention have in *vitro* activity as inhibitors of the enzyme phosphodiesterase with minimum values of CI₅₀ under 100 nM. the inhibiting activity in *vitro* was measured using methodologies known in the state of the art (*Bioorg. Med. Chem., 9, 3013,* 2001).

The following data illustrate the inhibiting activity of the compounds of the present invention on the enzyme PDE-5.

| Compound | CI_{5C} (nM) |
|---|---|
| **1** | 4.2 |
| **2** | 6.4 |
| **3** | 2.8 |
| **4** | 1.8 |
| **5** | 3.5 |
| **6** | 2.2 |
| **7** | 1.2 |

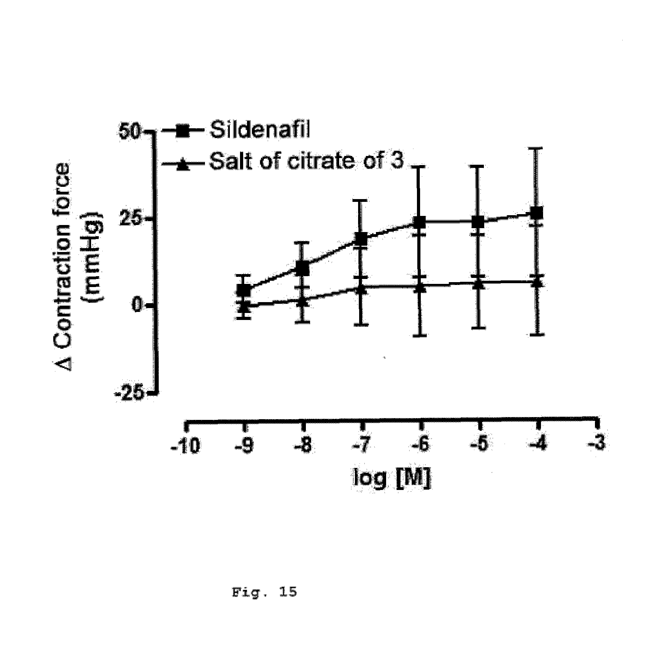

In addition, a comparative study was performed for the inotropic effect of sildefanil and the compound of general formula **I,** specifically compound **3,** on the left ventricle of a rat's heart, with a perfusion pressure of 140 mmHg (Langendorff model), finding an unexpected benefit as the citrate salt of the compound of formula **3** did not present a significant change in the contraction force in the left ventricle, unlike the cardiac wear caused by sildenafil, which is significant (**Figure 15**).

The invention has been described in sufficient detail for a person skilled in the art to reproduce it and obtain the results mentioned in the present patent application. However, any person skilled in the technological field of the present invention may be capable of making modifications to what is described in this application, so that if in order to apply such modifications in a specific procedure it is necessary to use the subject matter claimed in the appending claims, said procedure must be understood as falling within the scope of the present invention.

## Claims

1. A compound of general formula I 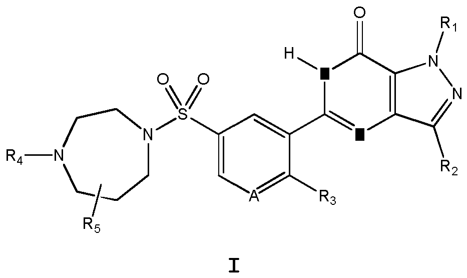 wherein:
A represents a carbon or nitrogen atom;
R₁ represents a proton, a linear or branched chain C₁-C₆ alkyl, a C₃-C₆ cycloalkyl, a COR₆ group, a derivate of carboxylic acid of C₁-C₈, a heterocyclic system with one ring or fused rings with three to six members in each ring, with one to five heteroatoms;
R₂ represents a proton, a linear or branched chain C₁-C₆ alkyl, a C₃-C₆ cycloalkyl or a C₁-C₃alkoxyl;
R₃ represents a proton, a linear or branched chain C₁-C₆ alkyl, a hydroxyl, a C₁-C₃ alkoxyl, optionally substituted, a halogen, a heterocyclic system with a single ring or fused rings with three to six members in each ring, with one to five heteroatoms;
R₄ represents a proton, a linear or branched chain C₁-C₈ alkyl optionally with a hydroxyl group at the end of the chain, a C₃-C₆ cycloalkyl optionally with a hydroxyl group in the ring, a derivate group of COR₇, a derivate group of CO₂R₈, a derivate of carboxylic acid with C₁-C₈ that can be fused directly to the nitrogen of the 1,4-diazepane or preceded by one, two or three methylene units, or an amine protector group;
R₅ represents a proton, a linear or branched chain C₁-C₆ alkyl, a functional group such as hydroxyl, methoxyl, or carboxylic acid;
R₆ represents a proton or a linear or branched chain C₁-C₆ alkyl;
R₇ represents a proton, a linear or branched chain C₁-C₆ alkyl, a C₃-C₆ cycloalkyl where it can be a saturated or aromatic ring, and can also contain a heterocycle, partially or totally saturated or unsaturated;
R₈ represents a proton, a linear or branched chain C₁-C₆ alkyl, a C₃-C₆ cycloalkyl where the ring can be saturated or aromatic, and can also contain groups such as benzyl or methylfluorenyl;
and their salts, N-oxide compounds, enantiomers, diastereoisomers, isotopes and tautomeric forms.

2. A compound of general formula I according to claim 1, **characterized in that**:
A represents a carbon or nitrogen atom;
R₁ represents a C₁-C₂ alkyl or a derivate of pentanoic acid;
R₂ represents a linear or branched chain C₁-C₃ alkyl;
R₃ represents a hydroxyl or alkoxyl group of C₁-C₃ optionally substituted;
R₄ represents a proton, a C₁-C₃ alkyl group optionally substituted with a hydroxyl group at the end of the chain, a COR₇ or an amine protector group;
R₅ represents a proton or a C₁-C₃ alkyl group;
R₇ represents a proton or a methyl;
and their salts, N-oxide compounds, enantiomers, diastereoisomers, isotopes and tautomeric forms.

3. A compound of general formula **I** according to claim 1, **characterized in that**:
A represents a carbon or nitrogen atom;
R₁ represents methyl or ethyl;
R₂ represents n-propyl;
R₃ represents ethoxyl or propoxyl;
R₄ represents a proton or methyl or ethyl or propyl, optionally substituted with a hydroxyl group, a COR₇ group or an amine protector group;
R₅ represents a proton or methyl;
R₇ represents a proton or methyl;
and their salts, N-oxide compounds, enantiomers, diastereoisomers, isotopes and tautomeric forms.

4. A compound of general formula **I** according to any O claims 1 to 3, **characterized in that** the compound is selected from among the group comprising:
5-(5-(1,4-diazepan-1-ylsulfonyl)-2-ethoxyphenyl)-1-methyl-3-propyl-1*H*-pyrazolo[4,3-*d*]pyrimidin-7(6*H*)-one;
5-(5-(4-acetyl-1,4-diazepan-1-ylsulfonyl)-2-ethoxyphenyl)-1-methyl-3-propyl-1*H*-pyrazolo[4,3-*d*]pyrimidin-7(6*H*)-one;
5-(2-ethoxy-5-(4-methyl-1,4-diazepan-1-ylsulfonyl)phenyl)-1-methyl-3-propyl-1*H*-pyrazolo[4,3-*d*]pyrimidin-7(6*H*)-one;
5-(2-ethoxy-5-(4-(2-hydroxypropyl)-1,4-diazepan-1-ylsulfonyl)phenyl)-1-methyl-3-propyl-1*H*-pyrazolo[4,3-*d*]pyrimidin-7(6*H*)-one;
tert-butyl 4-(4-ethoxy-3-(1-methyl-7-oxo-3-propyl-6,7-dihydro-1*H*-pyrazolo[4,3-*d*]pyrimidin-5-yl) phenylsulfonyl)-1,4-diazepane-1-carboxylate;
5-(2-ethoxy-5-(4-methyl-1,4-diazepan-1-ylsulfonyl)pyridin-3-yl)-1-methyl-3-propyl-1*H*-pyrazolo[4,3-*d*] pyrimidin-7(6*H*)-one;
or
2-(4-(4-ethoxy-3-(1-methyl-7-oxo-3-propyl-6,7-dihydro-1*H-*pyrazolo[4,3-d]pyrimidin-5-yl) phenylsulfonyl)-1,4-diazepan-1-yl) propionic acid,
and their salts, *N*-oxide compounds, enantiomers, diastereoisomers, isotopes and tautomeric forms.

5. A method for manufacture the compound of general formula **I** as claimed in claim 1, **characterized in that** it comprises the stages:
a) reacting a compound of general formula **II'** 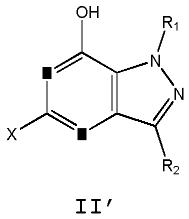 wherein:
R₁ and R₂ have been defined previously for the compound of general formula **I,** and X represents a halogen atom, such as fluorine, chlorine, bromine or iodine, with a metal such as magnesium or with zinc chloride (ZnCl₂), to form the compound of general formula **III'** 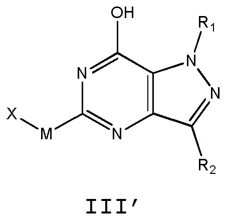 wherein:
R₁, R₂ and X have the meaning given above, and M represents an atom of magnesium or zinc;
b) performing a coupling reaction between the compound of general formula **III'** and the aryl halide of general formula **IV** 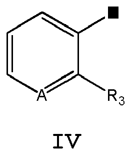 wherein:
A and R₃ have been defined previously for the compound of general formula **I,** and X represents a halogen atom such as fluorine, chlorine, bromine or iodine, in the presence of a suitable catalyst selected from among:
Ni(dppp)Cl₂ or Ni(PPh₃)₂Cl₂ complexes (Kharasch coupling conditions) for the case of the compound of general formula **III'** where M is an atom of magnesium,
or
metallic complexes of triphenylphosphines of palladium or nickel (Negishi coupling conditions) for the case of the compound of general formula **III'** where M is an atom of zinc, forming the compound of general formula, **V'** 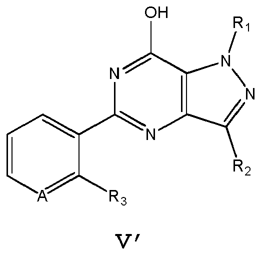 wherein:
A, R₁, R₂ and R₃ represent functional groups that have been defined previously for the compound of general formula **I;**
c) the compound of general formula **V' is** reacted with chlorosulfonic acid (ClSO₃H) in the presence of inert solvents and a base, to form the compound of general formula **VI'** 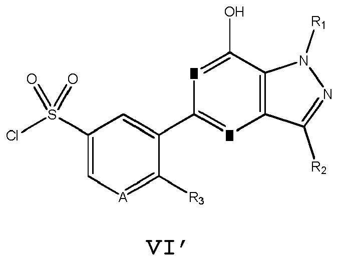 wherein:
A, R₁, R₂ and R₃ have been defined previously for the compound of general formula **I;**
and,
d) the compound of general formula **VI'** is reacted with 1,4-diazepane derivates of general formula **VII** 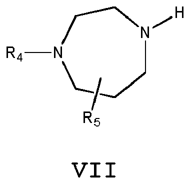 wherein:
R₄ and R₅ have been defined previously for the compound of general formula **I,**
in the presence of a solvent and a base, to form the compound of general formula **I'** 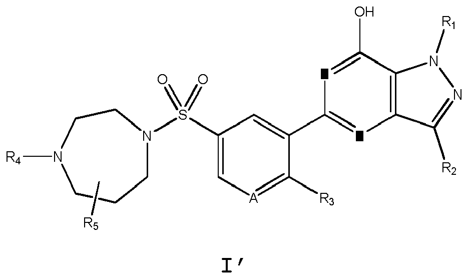
wherein:
A, R₁, R₂, R₃ R₄ and R₅ represent groups that have been defined previously for the compound of general formula **I,** wherein the compound of general formula **I'** is in iminol-amide tautomeric equilibrium with the compound of general formula **I.**

6. A method for manufacture the compound of general formula **I** as claimed in claim 1, **characterized in that** it comprises the stages:
a) reacting the compound of general formula **III'** 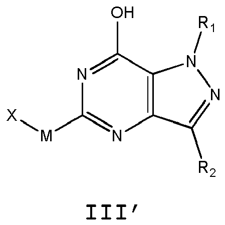 wherein:
R₁ and R₂ have the meaning given above for the compound of general formula **I,** X represents a halogen atom such as fluorine, chlorine, bromine or iodine, and M represents an atom of magnesium or zinc,
with a chlorosulfonated aryl halide of general formula **VIII,** 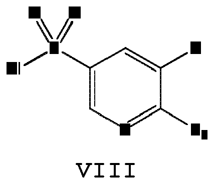
wherein:
A and R₃ have been defined previously for the compound of general formula **I,** and X has the meaning given above, performing the reaction in the presence of a catalyst selected from among:
Ni(dppp)Cl₂ or Ni(PPh₃)₂Cl₂ complexes (Kharasch coupling conditions) for the case of the compound of general formula **III'** where M is an atom of magnesium,
or
metallic complexes of triphenylphosphines of palladium or nickel (Negishi coupling conditions) for the case of the compound of general formula **III'** where M is an atom of zinc, forming the compound of general formula **VI'** 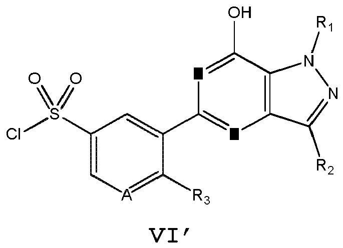
wherein:
A, R₁, R₂ and R₃ represent functional groups that have been defined previously for the compound of general formula **I;** and,
b) the compound of general formula **VI'** is reacted with 1,4-diazepane derivates of general formula **VII,** 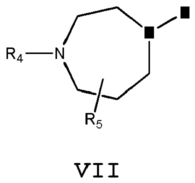 wherein:
R₄ and R₅ have been defined previously for the compound of general formula **I,** in the presence of a solvent and a base, to form the compound of general formula **I',** 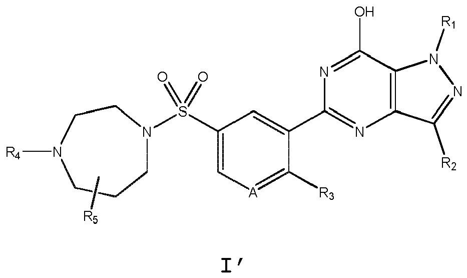
wherein:
A, R₁, R₂, R₃ R₄ and R₅ represent functional groups that have been defined previously for the compound of general formula **I,** where the compound of general formula **I'** is in iminol-amide tautomeric equilibrium with the compound of general formula **I.**

7. A method for manufacture the compound of general formula **I** as claimed in claim 1, **characterized in that** it comprises the stage:
a) reacting a compound of general formula **III',** 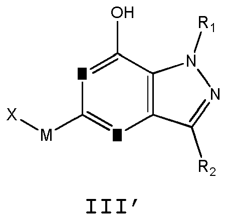 wherein:
R₁ and R₂ have the meaning given above for the compound of general formula **I,** X represents a halogen atom, such as fluorine, chlorine, bromine or iodine, and M represents an atom of magnesium or zinc, with an arylhalogenated sulfonamide of general formula **IX,** 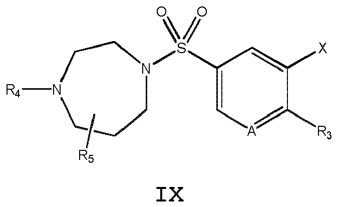 wherein:
A, R₃, R₄ and R₅ have been defined previously for the compound of general formula **I,** and X has the meaning given above, performing the reaction in the presence of a catalyst selected from:
Ni(dppp)Cl₂ or Ni(PPh₃)₂Cl₂ complexes (Kharasch coupling conditions) for the case of the compound of general formula **III'** where M is an atom of magnesium,
or
metallic complexes of triphenylphosphines of palladium or nickel (Negishi coupling conditions) for the case of the compound of general formula **III'** wherein M is an atom of zinc, forming the compound of general formula **I'** 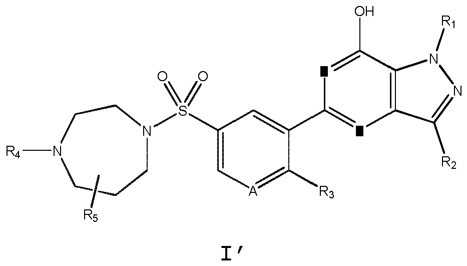
wherein:
A, R₁, R₂, R₃ R₄ and R₅ represent functional groups that have been defined previously for the compound of general formula **I,** where the compound of general formula **I'** is in iminol-amide tautomeric equilibrium with the compound of general formula **I.**

8. A method according to any one of claims 5 to 7, **characterized in that** it additionally comprises the stage of performing an acylation reaction on the obtained compound of formula **I** where R₄ is a proton, to obtain a compound of general formula **Ib,** 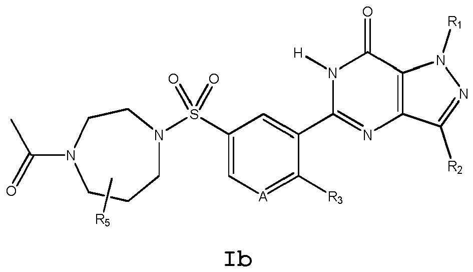 wherein:
A, R₁, R₂, R₃ and R₅ represent functional groups that have been defined for the compound of general formula **I.**

9. A method according to claim 8, further **characterized in that** the acylation reaction is performed:
i) with acid chloride in the presence of a base; or
ii) with acetic acid in the presence of a coupling agent.

10. A method according to any one of claims 5 to 7, **characterized in that** it additionally comprises the stage of performing an alkylation reaction on the obtained compound of formula **I** where R₄ is a proton, with alkyl halides of general formula **X,** 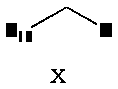 wherein:
R₁₀ represents a proton, or an linear or branched chain alkyl of up to seven carbon atoms, optionally substituted with hydroxyl groups or carboxylic acid at the end of the chain, and X represents a halogen atom, to obtain the compound of general formula **Ic,** 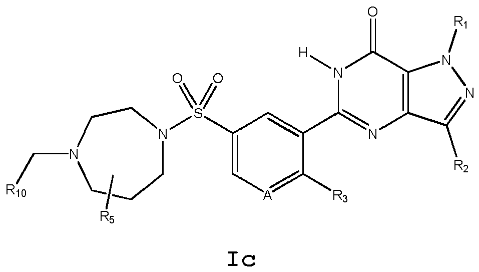 wherein:
A, R₁, R₂, R₃ and R₅ represent functional groups that have been defined previously for the compound of general formula **I.**

11. A pharmaceutical composition **characterized in that** it comprises a compound of general formula **I,** according to claim 1, and a coadjuvant agent, diluent agent, and/or pharmaceutically acceptable carrier.

12. A pharmaceutical composition according to claim 11, **characterized in that** it is formulated for administration by oral, buccal, sublingual, parenteral, topical, transdermal, tracheal, bronchial, nasal, pulmonary or rectal route.

13. A pharmaceutical composition according to claim 12, **characterized in that** it is in a pharmaceutical dosage form selected from tablets, chewable tablets, capsules, hard and soft gelatin capsules (including microcapsules), pills, solutions, parenteral solutions (creams and gels), syrups, concentrated drops, suppositories, suspensions, patches, pharmaceutical packets and powder for reconstitution or addition to food.

14. A pharmaceutical composition according to claim 13, **characterized in that** the dosage form is selected from tablets, pharmaceutical packets and powder to be reconstituted as syrup.

15. A pharmaceutical composition according to claim 11, **characterized in that** it also comprises an agent that stimulates the production of nitric oxide.

16. A pharmaceutical composition according to claim 15, **characterized in that** said stimulating agent is selected from the group comprising calcium dobesilate, coenzyme Q, L-carnitine and/or L-arginine and their structural analogues.

17. A pharmaceutical composition according to claim 11, **characterized in that** it also comprises a pharmaceutically acceptable polymer that controls the rate of release of the compound of general formula **I** to modify its release profile.

18. A pharmaceutical composition according to claim 17, **characterized in that** the rate of release is immediate, delayed or controlled.

19. The use of the compound of general formula **I** as defined in claim 1, in the preparation of a medicament useful in the prophylaxis and/or treatment of diseases caused by metabolic deficiency of cyclic guanosine-3',5'-monophosphate (cGMP) regulated by phosphodiesterase type enzymes.

20. The use according to claim 19, wherein the phosphodiesterase is enzyme PDE-5.

21. The use according to claim 19, wherein the disease is erectile dysfunction.
